# FASCICULE DE BREVET EUROPEEN

(11) **EP 1 043 344 B1**
(45) Date de publication et mention de la délivrance du brevet: **03.09.2003**
(21) Numéro de dépôt: 00400547.6
(22) Date de dépôt: 29.02.2000
(51) Int. Cl.: C08F 293/00, C08G 81/02, A61K 7/06, A61K 7/043, A61K 7/02

(54) **Composition cosmétique comprenant des polymères ayant une structure en étoiles et leur utilisation**
Zusammensetzungen für Kosmetik enthaltend sternförmige Polymerisate und ihre Anwendung
Cosmetic composition containing star shaped polymers and their use

(30) Priorité: 06.04.1999 FR 9904254
(43) Date de publication de la demande: 11.10.2000
(73) Titulaire: L'OREAL, 75008 Paris (FR)
(72) Inventeur: Mougin, Nathalie, 75011 Paris (FR)
(74) Mandataire: Dodin, Catherine

(56) Documents cités:
- WO-A-86/00626
- DE-A- 4 328 004
- DE-A- 19 602 540
- US-A- 3 907 984
- US-A- 5 804 664

## Description

La présente invention a trait à une composition, notamment cosmétique ou pharmaceutique, comprenant dans un milieu adéquat au moins un polymère de structure ordonnée bien particulière. Ces compositions trouvent une application particulière dans le domaine du maquillage et sont susceptibles d'être appliquées sur la peau, les semi-muqueuses et/ou les muqueuses.

Les compositions à appliquer sur la peau, les semi-muqueuses et/ou les muqueuses telles que les rouges à lèvres et les fonds de teint, se présentent généralement sous forme de stick, de pâte souple ou de pâte coulée, et comprennent des corps gras tels que des huiles, des composés pâteux et/ou des cires, et une phase particulaire généralement composée de charges et de pigments.
Certaines de ces compositions présentent toutefois l'inconvénient de transférer. On entend par là que la composition est susceptible de se déposer, au moins en partie, sur certains supports avec lesquels elle est mise en contact, tels que, par exemple, un verre, une tasse, un vêtement ou la peau. En se déposant, ladite composition laisse une trace sur ledit support. Il s'en suit donc une persistance médiocre de la composition sur la peau, les semi-muqueuses ou les muqueuses, et la nécessité de renouveler régulièrement son application.
Par ailleurs, l'apparition de traces inacceptables sur certains vêtements et notamment sur les cols de chemisier peut écarter certaines femmes de l'utilisation de ce type de maquillage.
Un autre inconvénient de ces compositions réside dans le problème de migration. On a en effet constaté que certaines compositions avaient tendance à se propager à l'intérieur des ridules et/ou des rides de la peau, dans le cas des fonds de teint; dans les ridules qui entourent les lèvres, dans le cas des rouges à lèvres; dans les plis de la paupière, dans le cas des fards à paupières. On a également constaté, dans le cas notamment des fards à paupières, l'apparition de stries dans le maquillage, générées par les mouvements des paupières. On a encore constaté que les eye-liners pouvaient également couler. Tous ces phénomènes engendrent un effet inesthétique que l'on souhaite bien évidemment éviter.

Depuis plusieurs années, de nombreux cosméticiens se sont intéressés aux compositions cosmétiques, notamment de rouge à lèvres ou de fond de teint 'sans transfert'. Ainsi, il a été envisagé des compositions liquides de rouge à lèvres 'sans transfert' contenant de 1 à 70% en poids de résine de silicone à motifs répétitifs silicates, de 10 à 98% en poids d'une huile de silicone volatile et des charges pulvérulentes. Toutefois, le film obtenu sur les lèvres après évaporation de l'huile de silicone présente l'inconvénient de devenir inconfortable au cours du temps (sensation de dessèchement et de tiraillement).
On connaît également des rouges à lèvres 'sans transfert' contenant une silicone volatile et une résine de silicone liquide comportant une chaîne estérifiée pendante ayant au moins 12 atomes de carbone. Le film de rouge à lèvres présente notamment l'inconvénient de manquer de confort à l'application, en particulier d'être trop sec.
D'autre part, il a été décrit des compositions cosmétiques de maquillage comprenant des polymères filmogènes en solution aqueuse; mais ces compositions sont sensibles à l'eau et ne peuvent donc pas, notamment, être appliquées sur les lèvres. Lorsque les polymères sont solubilisés dans des milieux alcooliques ou hydroalcooliques, on a constaté que la composition obtenue pouvait engendrer des problèmes d'irritation et/ou de déshydratation de la peau, d'où un inconfort certain pour l'utilisatrice.
On connaît également, par exemple par le document EP820764, une composition susceptible d'être appliquée sur la peau, les semi-muqueuses et/ou les muqueuses, comprenant, dans un système polymérique, une dispersion aqueuse de particules de polymère filmogène, ledit système polymérique permettant l'obtention d'un film ayant un module de Young inférieur à environ 200 MPa.
Par ailleurs, on connaît, par le document WO86/00626 des polymères étoiles de type acryliques, susceptibles d'être employés notamment comme agent renforçateur dans les domaines des matériaux composites, des matériaux plastiques, des revêtements pour automobiles ou camions.
On connaît également par le document US5804664 des polymères étoiles à branches multiples, comprenant au moins un polyisobutylène, et pouvant être employés comme agent modificateur de viscosité ou comme additif pour les huiles de moteur.
On connaît encore par DE4328004 des copolymères blocs, notamment diblocs, à base de polyméthacrylates et/ou de polyacrylates, qui trouvent une application dans le domaine thermoplastique.

La présente invention a pour but de proposer une composition qui ne présente pas les inconvénients de l'art antérieur, et qui soit en particulier confortable à appliquer et à porter, tout en n'étant pas collante au toucher (absence de 'tack').
En effet, on a constaté que généralement les compositions destinées à être appliquées sur la peau, telles que les fonds de teint, les rouges à lèvres ou les compositions de soin, qui comprenaient des polymères filmogènes, ne pouvaient être à la fois confortables à porter et peu collantes au toucher, même après séchage.
Il revient à la demanderesse le mérite d'avoir mis en évidence qu'en utilisant les nouveaux polymères objets de la présente invention, il était possible d'obtenir un compromis cosmétiquement satisfaisant entre ces deux propriétés, tout en conservant aux compositions les qualités cosmétiques adéquates et attendues par la consommatrice.

Ainsi, un objet de la présent invention est une composition cosmétique de maquillage du visage ou du corps, comprenant, dans un milieu cosmétiquement acceptable défini, un polymère de structure en "étoiles" tel que défini ci-après.

Un autre objet de l'invention est un procédé de traitement cosmétique des matières kératiniques telles que la peau, les cils, les sourcils, les ongles, les lèvres, caractérisé en ce qu'il consiste à appliquer sur ces dernières une composition cosmétique telle que définie ci-dessus.

Un autre objet de l'invention est l'utilisation d'au moins un polymère tel que ci-dessous défini, dans une composition cosmétique pour diminuer, voire supprimer, le transfert du film de composition déposé.

Les compositions selon l'invention présentent une texture légère et sont très confortables à porter tout au long de la journée.
De plus, ces compositions permettent l'obtention d'un film de très bonne tenue, qui ne transfère pas et ne tache pas un support avec lequel il serait en contact, et qui ne migre pas au cours du temps.
Le film est mou, souple, élastique et flexible sur la peau; il suit les mouvements du support sur lequel il est déposé, sans se craqueler et/ou se décoller. Il adhère notamment parfaitement sur les lèvres du visage.
Un autre avantage apporté par la présente invention est qu'il est possible d'obtenir un film sans transfert non collant et confortable.
La composition selon l'invention est facilement applicable et s'étale aisément en particulier sur les lèvres du visage. La composition selon l'invention trouve notamment une application particulièrement intéressante dans le domaine du maquillage de la peau, des muqueuses et/ou des semi-muqueuses de l'être humain.

On entend notamment par muqueuse, la partie interne de la paupière inférieure; parmi les semi-muqueuses, on entend plus particulièrement les lèvres du visage.
Ainsi, la composition selon l'invention trouve une application préférée dans le domaine des produits de maquillage des lèvres du visage, notamment en tant que rouge à lèvres. Elle trouve également une autre application avantageuse dans le domaine des fonds de teint ou des fards à joues ou à paupières.

La composition selon l'invention comprend donc un polymère dont la structure en "étoiles" peut être illustrée, de manière générale, par la formule suivante (I) :

A-[(M1)ₚ₁ - (M2)ₚ₂ .... (Mi)ₚⱼ]ₙ

dans laquelle :
- A représente un centre multifonctionnel, de fonctionnalité "n", n étant un entier supérieur à 2, de préférence compris entre 4 et 10,
- [(M1)ₚ₁ - (M2)ₚ₂ .... (Mi)ₚⱼ] représente une chaîne polymérique, aussi appelée "branche", constituée de monomères Mi polymérisés, identiques ou différents, ayant un indice de polymérisation pj, chaque branche étant identique ou différente, et étant greffée de manière covalente sur ledit centre A,
- i étant supérieur ou égal à 2, de préférence compris entre 2 et 10;
- pj étant supérieur ou égal à 2, de préférence compris entre 10 et 20 000.

De préférence, les chaînes polymériques se présentent sous forme de blocs, de masse moléculaire supérieure ou égale à 500, pouvant aller jusqu'à 2 000 000.

Dans un mode de réalisation préférée, le polymère utilisé dans le cadre de la présente invention peut être obtenu par polymérisation radicalaire contrôlée, également appelée polymérisation radicalaire "vivante". Cette technique permet notamment de surmonter les limitations inhérentes à la polymérisation radicalaire classique, c'est-à-dire qu'elle permet notamment de contrôler la longueur des chaînes du polymère formé, et de ce fait d'obtenir des structures blocs.
La polymérisation radicalaire contrôlée permet de réduire les réactions de désactivation de l'espèce radicalaire en croissance, en particulier l'étape de terminaison, réactions qui, dans la polymérisation radicalaire classique, interrompent la croissance de la chaîne polymérique, de façon irréversible et sans contrôle.
Afin de diminuer la probabilité des réactions de terminaison, il a été proposé de bloquer, de façon transitoire et réversible, l'espèce radicalaire en croissance, en formant des espèces actives dites "dormantes" à l'aide de liaison de faible énergie de dissociation.

On utilise des liaisons de type C-halogénure (en présence de complexe métal/ligand). On parle alors de polymérisation radicalaire par transfert d'atomes, aussi connue sous l'abréviation ATRP. Ce type de polymérisation se traduit par un contrôle de la masse des polymères formés et par un faible indice de polydispersité en poids des chaînes.
D'une manière générale, la polymérisation radicalaire par transfert d'atomes s'effectue par polymérisation:
- d'un ou plusieurs monomères polymérisables radicalairement, en présence
- d'un initiateur ayant au moins un atome ou un groupe radicalairement transférable,
- d'un composé comprenant un métal de transition, susceptible de participer à une étape de réduction avec l'initiateur et une chaîne polymérique "dormante", et
- d'un ligand, pouvant être choisi parmi les composés comprenant un atome d'azote (N), d'oxygène (O), de phosphore (P) ou de soufre (S), susceptibles de se coordonner par une liaison σ audit composé comprenant un métal de transition, ou parmi les composés comprenant un atome de carbone susceptibles de se coordonner par une liaison π ou σ audit composé comprenant un métal de transition, la formation de liaisons directes entre ledit composé comprenant un métal de transition et le polymère en formation étant évitées.
   Ce procédé est en particulier illustré dans la demande WO97/18247, dont l'homme du métier pourra tirer enseignement pour préparer les polymères entrant dans le cadre de la présente invention.

La nature et la quantité des monomères, initiateur(s), composé(s) comprenant le métal de transition et ligand(s) seront choisies par l'homme du métier sur la base de ses connaissances générales, en fonction du résultat recherché.

En particulier, les monomères "M" peuvent être choisis, seuls ou en mélange, parmi les composés à insaturation éthylénique, radicalairement polymérisables, répondant à la formule : 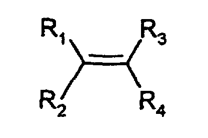 dans laquelle R₁, R₂, R₃ et R₄ sont, indépendamment les uns des autres, choisis parmi:
- un atome d'hydrogène;
- un atome d'halogène;
- un radical alkyle, linéaire ou ramifié, ayant 1 à 20, de préférence 1-6, plus préférentiellement 1-4, atomes de carbone, éventuellement substitué par un ou plusieurs halogènes et/ou un ou plusieurs radicaux -OH;
- un radical alcényle ou alkynyle, linéaire ou ramifié, ayant 2 à 10, de préférence 2-6, plus préférentiellement 2-4, atomes de carbone, éventuellement substitué par un ou plusieurs halogènes;
- un radical hydrocarboné cyclique (cycloalkyle) ayant 3 à 8 atomes de carbone, éventuellement substitué par un ou plusieurs atomes d'halogène, d'azote, de soufre, d'oxygène;
- un radical choisi parmi CN, C(=Y)R⁵, C(=Y)NR⁶R⁷, YC(=Y)R⁵, NC(=Y)R⁵ cyclique, SOR⁵, SO₂R⁵, OSO₂R⁵, NR⁸SO₂R⁵, PR⁵₂, P(=Y)R⁵₂, YPR⁵₂, YP(=Y)R⁵₂, NR⁸₂ qui peut être quatemisé avec un groupe additionnel R⁸, aryle et hétérocyclyle.
   avec :
   - Y représente O, S ou NR⁸ (de préférence O),
   - R⁵ représente un radical alkyle, alkylthio, alcoxy, linéaire ou ramifié, ayant 1-20 atomes de carbone; un radical OH; un radical OM' avec M' = métal alcalin; un radical aryloxy ou un radical heterocyclyloxy;
   - R⁶ et R⁷ représentent, indépendamment l'un de l'autre, H ou un radical alkyle, linéaire ou ramifié, ayant 1-20 atomes de carbone; étant donné que R⁶ et R⁷ peuvent être joints pour former un groupe alkylène ayant 2-7, de préférence 2-5, atomes de carbone;
   - R⁸ représente H; un radical alkyle, linéaire ou ramifié, ayant 1-20 atomes de carbone ou un radical aryle;
- un radical -COOR dans lequel R est un radical alkyle, linéaire ou ramifié, ayant 1 à 20, de préférence 1-6, atomes de carbone, éventuellement substitué par un ou plusieurs halogènes;
- un radical -CONHR' dans lequel R' est l'hydrogène ou un radical hydrocarboné, saturé ou insaturé, linéaire ou ramifié, ayant 1 à 20, de préférence 1-6, atomes de carbone, éventuellement substitué par un ou plusieurs halogènes, azotes et/ou oxygènes;
- un radical -OCOR" dans lequel R" est l'hydrogène ou un radical hydrocarboné, saturé ou insaturé, linéaire ou ramifié, ayant 1 à 20 atomes de carbone, éventuellement substitué par un ou plusieurs halogènes, azotes et/ou oxygènes;
- un radical comprenant au moins un atome de silicium, et notamment des radicaux tels que : un radical -R-siloxane; un radical -CONHR-siloxane; un radical -COOR-siloxane ou un radical -OCO-R-siloxane, dans lesquels R est un radical alkyle, alkylthio, alcoxy, aryloxy ou hétérocycloxy, linéaire ou ramifié, ayant 1-20 atomes de carbone.

Par siloxane, on entend un composé comprenant des motifs (-SiR^{a}R^{b}O-)n, dans lesquels R^{a} et R^{b} peuvent représenter, indépendamment l'un de l'autre, un hydrogène; un halogène; un radical hydrocarboné, linéaire ou ramifié, saturé ou insaturé, ayant 1 à 36 atomes de carbone, éventuellement substitué par un ou plusieurs halogènes, azotes et/ou oxygènes; un radical hydrocarboné cyclique ayant 1 à 20 atomes de carbone; n étant supérieur ou égal à 1.
Notamment on peut citer les polydiméthylsiloxanes (PDMS) comprenant 1 à 200, de préférence moins de 100, motifs de répétition.

Par ailleurs, R¹ et R³ peuvent être reliés entre eux de manière à former un cycle de formule (CH₂)n, qui peut être substitué par un ou plusieurs halogènes et/ou oxygènes et/ou azote, et/ou par des radicaux alkyles ayant 1 à 6 atomes de carbone.

Par 'aryle' ou 'hétérocyclyle' on entend la définition communément comprise par l'homme du métier et qui peut être illustrée par l'art antérieur WO97/18247.

De préférence, on peut choisir les monomères M dans le groupe constitué par :
- les esters acryliques ou méthacryliques obtenus à partir d'alcools aliphatiques, linéaires, ramifiés, cycliques et/ou d'alcools aromatiques, de préférence en C₁-C₂₀ tel que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyle ;
- les (méth)acrylates d'hydroxyalkyle en C₁-C₄ tels que le (méth)acrylate de 2-hydroxyéthyle ou le (méth)acrylate de 2-hydroxypropyle;
- les (méth)acrylates d'éthylèneglycol, de diéthylèneglycol, de polyéthylèneglycol, à extrémité hydroxyle ou éther;
- les esters vinyliques, allyliques ou méthallyliques obtenus à partir d'alcools aliphatiques, linéaires ou ramifiés en C₁-C₁₀ ou cycliques en C₁-C₆ et/ou d'alcools aromatiques, de préférence en C₁-C₆, tels que l'acétate de vinyle, le propionate de vinyle, le benzoate de vinyle, le tertio-butyl benzoate de vinyle ;
- la N-vinylpyrrolidone; la vinylcaprolactame; les vinyl N-alkylpyrroles ayant 1 à 6 atomes de carbone; les vinyl-oxazoles; les vinyl-thiazoles; les vinylpyrrimidines; les vinylimidazoles; les vinyl cétones;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄) (méth)acrylamides ;
- les oléfines tels que l'éthylène, le propylène, le styrène ou le styrène substitué;
- les monomères acryliques ou vinyliques fluorés ou perfluorés, notamment les esters (méth)acryliques à motifs perfluoroalkyle;
- les monomères comportant une fonction amine sous forme libre ou bien partiellement ou totalement neutralisée ou bien partiellement ou totalement quaternisée tels que le (méth)acrylate de diméthylaminoéthyle, le diméthylaminoéthyl méthacrylamide, la vinylamine, la vinylpyridine, le chlorure de diallyldiméthylammonium;
- les carboxybétaïnes ou les sulfobétaïnes obtenues par quatemisation partielle ou totale de monomères à insaturation éthylénique comportant une fonction amine par des sels de sodium d'acide carboxylique à halogénure mobile (chloroacétate de sodium par exemple) ou par des sulfones cycliques (propane sulfone);
- les (méth)acrylates ou (méth)acrylamides siliconés, notamment les esters(méth)acryliques à motifs siloxane;
- leurs mélanges.

Les monomères particulièrement préférés sont choisis parmi :
- les esters (méth)acryliques obtenus à partir d'alcools aliphatiques, linéaires ou ramifiés, de préférence en C₁-C₂₀;
- les esters (méth)acryliques en C₁-C₂₀ à motifs perfluoroalkyle;
- les esters(méth)acryliques en C₁-C₂₀ à motifs siloxane;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄) (méth)acrylamides ;
- les esters vinyliques, allyliques ou méthallyliques obtenus à partir d'alcools aliphatiques, linéaires ou ramifiés en C₁-C₁₀ ou cycliques en C₁-C₆;
- la vinylcaprolactame ;
- le styrène éventuellement substitué;
- leurs mélanges.

Dans le cadre de la présente invention, l'initiateur peut être tout composé, notamment moléculaire ou polymérique, ayant au moins deux atomes et/ou groupes radicalairement transférables par polymérisation.
Notamment, l'initiateur peut être un oligomère ou un polymère susceptible d'être obtenu par polymérisation radicalaire, par polycondensation, par polymérisation anionique ou cationique, ou par ouverture de cycles.
Lesdits atomes et/ou groupes transférables peuvent être situés aux extrémités de la chaîne polymérique ou le long du squelette.

En particulier, on peut citer les composés correspondant à l'une des formules suivantes :
- R¹¹ₓ R¹²_{y} R¹³_{z} C-(RX)ₜ dans laquelle x, y et z représentent un entier allant de 0 à 4, t un entier allant de 1 à 4, et x+y+z= 4-t;
- R¹³ₓ C₆-(RX)_{y} (cycle à 6 carbones, saturé) dans laquelle x représente un entier allant de 7 à 11, y représente un entier allant de 1 à 5, et x+y= 12 ;
- R¹³ₓ C₆-(RX)_{y} (cycle à 6 carbones, insaturé) dans laquelle x représente un entier allant de 0 à 5, y représente un entier allant de 1 à 6, et x+y= 6;
- -[-(R¹¹)(R¹²)(R¹³)C-(RX)-]ₙ dans laquelle n est supérieur ou égal à 1; cyclique ou linéaire;
- -[-(R¹²)ₓ C₆ (RX)_{y}- R¹¹-]ₙ dans laquelle x représente un entier allant de 0 à 6, y représente un entier allant de 1 à 6, et n est supérieur ou égal à 1, avec x+y=4 ou 6; cyclique ou linéaire;
- -[-(R¹²)ₓ C₆ (RX)y- R¹¹-]ₙ dans laquelle x représente un entier allant de 0 à 12, y représente un entier allant de 1 à 12, et n est supérieur ou égal à 1, avec x+y=10 ou 12; cyclique ou linéaire;
- -[OSi (R¹¹)ₓ(RX)_{y}]ₙ, cyclique ou linéaire, dans laquelle x et y représentent un entier allant de 0 à 2, et n est supérieur ou égal à 1, avec x+y=2;
- R¹¹N-X₂
- (R¹¹)ₓP(O)_{y}-X₃₋ₓ dans laquelle x et y représentent des entiers allant de 0 à 2, et x+y = 5;
- (R¹¹O)ₓP(O)_{y}-X₃₋ₓ dans laquelle x et y représentent des entiers allant de 0 à 2, et x+y = 5;
- -[(R¹¹)ₜN_{z}P(O)ₓ(O-RX)_{y}-]ₙ, cyclique ou linéaire, dans laquelle x représente un entier allant de 0 à 4, y représente un entier allant de 1 à 5, z représente un entier allant de 0 à 2 et t représente un entier allant de 0 à 3, et n est supérieur ou égal à 1;
dans lesquelles :
- R, R¹¹, R¹² et R¹³ représentent, indépendamment l'un de l'autre, un atome d'hydrogène ou d'halogène; un radical alkyle, linéaire ou ramifié, ayant 1-20, de préférence 1-10 et plus préférentiellement 1-6 atomes de carbone; un radical cycloalkyle ayant 3-8 atomes de carbone; un radical -C(=Y)R⁵, -C(=Y)NR⁶R⁷ ou -R⁸₃Si (voir les définitions de R⁵ à R⁸ ci-dessus); -COCI, -OH, -CN, un radical alkényle ou alkynyle ayant 2-20, de préférence 2-6, atomes de carbone; un radical oxiranyle, glycidyle, alkylène ou alkénylène substitué avec un oxiranyle ou un glycidyle; un radical aryle, heterocyclyle, aralkyle, aralkenyle; un radical alkyle ayant 1-6 atomes de carbone dans lequel tout ou partie des atomes d'hydrogène sont substitués soit par des atomes d'halogènes tels que fluor, chlore ou brome, soit par un groupe alcoxy ayant 1-4 atomes de carbone ou par un radical aryle, heterocyclyle, -C(=Y)R⁵, -C(=Y)NR⁶R⁷, oxiranyle, glycidyle;
- X représente un atome d'halogène tel que Cl, Br, I, ou un radical -OR', -SR, -SeR, -OC(=O)R', -OP(=O)R', -OP(=O)(OR')₂, -OP(=O)OR', -O-NR'₂, -S-C(=S)NR'₂, -CN, -NC, -SCN, -CNS, -OCN, -CNO et -N₃, dans lequel R' représente un radical alkyle ayant 1-20 atomes de carbone éventuellement substitué par un ou plusieurs atomes d'halogènes notamment de fluor et/ou de chlore; et R représente un radical aryle ou alkyle, linéaire ou ramifié, ayant 1-20, de préférence 1-10, atomes de carbone; le groupement -NR'₂ pouvant en outre représenter un groupement cyclique, les deux groupes R' étant joints de manière à former un hétérocycle à 5, 6 ou 7 membres.

De préférence, X représente un atome d'halogène et notamment un atome de chlore ou de brome.

De préférence, on choisit l'initiateur parmi les composés de formule
- R¹³ₓ C₆-(RX)_{y} (cycle à 6 carbones, saturé) dans laquelle x représente un entier allant de 7 à 11, y représente un entier allant de 1 à 5, et x+y= 12 ;
- -[-(R¹²)ₓ C₆(RX)_{y}- R¹¹-]ₙ dans laquelle x représente un entier allant de 0 à 6, y représente un entier allant de 1 à 6, et n est supérieur ou égal à 1, avec x+y=4 ou 6; cyclique ou linéaire; et
- -[OSi (R¹¹)ₓ(RX)_{y}]ₙ, cyclique ou linéaire, dans laquelle x et y représentent un entier allant de 0 à 2, et n est supérieur ou égal à 1, avec x+y=2.

En particulier, on peut citer comme initiateur, les composés suivants :
- l'octa-2-isobutyrylbromide-octatertiobutyl-calix(8)arène,
- l'octa-2-propionylbromide -octatertiobutyl-calix(8)arène, et
- l'hexakis α-bromométhylbenzène.

Le composé comprenant un métal de transition, susceptible de participer à une étape de réduction avec l'initiateur et une chaîne polymérique "dormante", peut être choisi parmi ceux qui correspondent à la formule Mⁿ⁺X'ₙ, dans laquelle :
- M peut être choisi parmi Cu, Au, Ag, Hg, Ni, Pd, Pt, Rh, Co, Ir, Fe, Ru, Os, Re, Mn, Cr, Mo, W, V, Nb, Ta et Zn,
- X' peut représenter un halogène (brome ou chlore notamment), OH, (O)_{1/2}, un radical alcoxy ayant 1-6 atomes de carbone, (SO₄)_{1/2}, (PO₄)_{1/3}, (HPO₄)_{1/2}, (H₂PO₄), un radical triflate, hexafluorophosphate, methanesulfonate, arylsulfonate, SeR, CN, NC, SCN, CNS, OCN, CNO, N₃ et R'CO₂, dans lequel R représente un radical aryle ou alkyle, linéaire ou ramifié, ayant 1-20, de préférence 1-10 atomes de carbone, et R' représente H ou un radical alkyle, linéaire ou ramifié, ayant 1-6 atomes de carbone, ou un radical aryle, éventuellement substitué par un ou plusieurs atomes d'halogènes notamment de fluor et/ou de chlore;
- n est la charge du métal.

De préférence, on choisit M représentant le cuivre ou le ruthénium, et X' représentant le brome ou le chlore.
En particulier, on peut citer le bromure de cuivre.

Parmi les ligands susceptibles d'être utilisés dans le cadre de la présente invention, on peut citer les composés comprenant au moins un atome d'azote, d'oxygène, de phosphore et/ou de soufre, susceptibles de se coordonner par une liaison σ au composé comprenant un métal de transition.
On peut aussi citer les composés comprenant au moins deux atomes de carbone susceptibles de se coordonner par une liaison π audit composé comprenant un métal de transition.
On peut encore citer les composés comprenant au moins un atome de carbone susceptibles de se coordonner par une liaison a audit composé comprenant un métal de transition, mais qui ne forment pas de liaison carbone-carbone avec le monomère lors de la polymérisation, c'est-à-dire qui ne participent pas à des réactions de β-addition avec les monomères.
On peut encore citer les composés susceptibles de se coordonner par une liaison µ ou η audit composé comprenant un métal de transition.

Notamment, on peut citer les composés de formule : R⁹-Z-(R¹⁴-Z)ₘ-R¹⁰
dans laquelle :
- R⁹ et R¹⁰ sont, indépendamment l'un de l'autre, un atome d'hydrogène; un radical alkyle, linéaire ou ramifié, ayant 1-20, de préférence 1-10 atomes de carbone; un radical aryle; un radical hétérocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸ (voir les définitions de R⁵ à R⁸ et Y ci-dessus);
   étant donné que R⁹ et R¹⁰ peuvent être joints de manière à former un cycle, saturé ou insaturé;
- R¹⁴ représente, indépendamment les uns des autres, un groupe divalent choisi parmi les alcanediyles ayant 2-4 atomes de carbone; les alkénylènes ayant 2-4 atomes de carbone; les cycloalcanediyles ayant 3-8 atomes de carbone; les cycloalkènediyles ayant 3-8 atomes de carbone; les arènediyles et les hétérocyclylènes;
- Z représente O, S, NR¹⁵ ou PR¹⁵ avec R¹⁵ représentant H; un radical alkyle, linéaire ou ramifié, ayant 1-20 atomes de carbone; un radical aryle; un radical heterocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸ (voir les définitions de R⁵ à R⁸ et Y ci-dessus);
- m est compris entre 0 et 6.

On peut également citer les composés de formule :

R²⁰R²¹C[C(=Y)R⁵]

dans laquelle :
- R²⁰ et R²¹ sont, indépendamment l'un de l'autre, un atome d'hydrogène; un atome d'halogène; un radical alkyle, linéaire ou ramifié, ayant 1-20, de préférence 1-10 atomes de carbone; un radical aryle; un radical hétérocyclyle; étant donné que R²⁰ et R²¹ peuvent être joints de manière à former un cycle, saturé ou insaturé; étant donné que chaque radical peut en outre être substitué avec un radical alkyle ayant 1-6 atomes de carbone, un radical alcoxy ayant 1-6 atomes de carbone ou un radical aryle;
- R⁵ et Y étant définis ci-dessus.

On peut encore citer comme ligands, le monoxyde de carbone; les porphyrines et les porphycènes, éventuellement substitués; l'éthylènediamine et le propylènediamine, éventuellement substitués; les multiamines avec amines tertiaires telles que le pentaméthyldiéthylènetriamine; les aminoalcools tels que l'aminoéthanol et l'aminoprpoanol, éventuellement substitués; les glycols tels que l'éthylèneglycol ou le propylèneglycol, éventuellement substitués; les arènes tels que le benzène, éventuellement substitués; le cyclopentadiène, éventuellement substitué; les pyridines et bipyridines, éventuellement substituées; l'acétonitrile; la 1,10-phénanthroline; les cryptands et les éthers-couronnes; la spartéine.

Les ligands préférés sont choisis notamment parmi les pyridines et bipyridines, éventuellement substituées par des radicaux alkyls en C2-C15, en particulier en C6-C12, et notamment le radical nonyle; les multiamines avec amines tertiaires telles que la pentaméthyldiéthylènetriamine.

La polymérisation des monomères, en présence de l'initiateur, du composé comprenant un métal de transition et du ligand qui joue le rôle d'activateur, conduit à l'obtention d'un polymère ayant une structure d'étoiles, qui peut être représentée par la formule (I) donnée ci-dessus, dans laquelle les monomères se sont polymérisés pour donner "n" chaînes polymériques, semblables ou différentes, toutes reliées à un centre multifonctionnel A qui dérive de l'initiateur.

On a constaté que pour atteindre le but poursuivi par la présente invention, c'est-à-dire obtenir une composition qui ne présente pas les inconvénients de l'art antérieur et qui soit en particulier confortable à appliquer et à porter, tout en n'étant pas collante au toucher (absence de 'tack'), il était nécessaire de choisir un polymère répondant aux critères suivants :
- il doit comprendre un ou plusieurs monomères Mi dont l'homopolymère correspondant présente une Tg supérieure ou égale à environ 0°C, de préférence supérieure ou égale à 5°C, et encore mieux supérieure ou égale à 10°C;
- ce ou ces monomères Mi étant présents, dans le polymère final, en une quantité minimale d'environ 40% en poids, de préférence en une quantité comprise entre 50 et 99% en poids, et encore mieux en une quantité de 60-90% en poids, par rapport au poids total de monomères ;
et
- le polymère doit par ailleurs comprendre un ou plusieurs monomères Mj dont l'homopolymère correspondant présente une Tg inférieure ou égale à environ 0°C, de préférence inférieure ou égale à -10°C, et encore mieux inférieure ou égale à -15°C,
- ce ou ces monomères Mj étant présents, dans le polymère final, en une quantité maximale d'environ 60% en poids, de préférence en une quantité comprise entre 1 et 50% en poids, et encore mieux en une quantité de 10-40% en poids, par rapport au poids total de monomères.

La mesure de Tg (température de transition vitreuse) est effectuée par DSC (Differential Scanning Calorimetry) selon la norme ASTM D3418-97.

Les polymères tels que définis dans la présente invention doivent être filmogènes ou peuvent être rendus filmogènes par addition d'un agent auxiliaire de filmification. Par filmogène, on entend que le polymère, après application sur un support et évaporation du solvant (aqueux ou organique) conduit à un film transparent et non craquelé.
Un tel agent auxiliaire de filmification peut être choisi parmi tous les composés connus de l'homme du métier comme étant susceptibles de remplir la fonction recherchée, et peut être notamment choisi parmi les agents plastifiants et/ou parmi les agents de coalescence. En particulier, on peut citer, seuls ou en mélange :
- les glycols et leurs dérivés tels que le diéthylène glycol éthyléther, le diéthylène glycol méthyléther, le diéthylène glycol butyléther, le diéthylène glycol hexyléther, l'éthylène glycol éthyléther, l'éthylène glycol butyléther, l'éthylène glycol hexyléther;
- les esters de glycérol, tels que le diacétate de glycérol (diacétine) et le triacétate de glycérol (triacétine);
- les dérivés de propylène glycol et en particulier le propylène glycol phényléther, le propylène glycol diacétate, le propylène glycol méthyléther, le propylène glycol éthyléther, le propylène glycol butyléther, le dipropylène glycol méthyléther, le dipropylène glycol butyléther, le dipropylène glycol éthyléther, le tripropylène glycol butyléther, le tripropylène glycol méthyléther;
- des esters d'acides notamment carboxyliques, tels que des citrates, des phtalates, des adipates, des carbonates, des tartrates, des phosphates, des sébaçates,
- des dérivés oxyéthylénés tels que les huiles oxyéthylénées, notamment les huiles végétales telles que l'huile de ricin; les huiles de silicone oxyéthylénées.
La quantité d'agent auxiliaire de filmification peut être choisie par l'homme du métier sur base de ses connaissances générales, de manière à un film ayant les propriétés mécaniques souhaitées, tout en conservant à la composition des propriétés cosmétiquement acceptables.

Dans un mode de réalisation préféré de l'invention, on choisit un polymère, éventuellement en association avec des agents auxiliaires de filmification, permettant l'obtention d'un film ayant au moins l'une des caractéristiques physicochimiques suivantes :
- un module d'élasticité (module de Young) inférieur à environ 200 MPa, de préférence compris entre 2 et 100 MPa, préférentiellement compris entre 5 et 80 MPa;
- une dureté inférieure à 110, de préférence comprise entre 1 et 70 et encore mieux comprise entre 5 et 55.
Les méthodes de mesure sont décrites avant les exemples.

Les polymères tels que ci-dessus définis peuvent être présents dans le milieu sous forme dissoute ou en dispersion, dans une phase aqueuse, organique ou hydroorganique notamment alcoolique ou hydroalcoolique, et/ou une phase grasse, selon l'application envisagée.
Lesdits polymères peuvent être présents dans les compositions selon l'invention en une quantité aisément déterminable par l'homme du métier selon l'application envisagée, et qui peut être comprise entre 1 et 50% en poids de matière sèche, par rapport au poids total de la composition, de préférence entre 1 et 40% en poids et préférentiellement entre 5 et 35% en poids.

Les compositions cosmétiques ou pharmaceutiques selon l'invention comprennent donc en outre un milieu cosmétiquement ou pharmaceutiquement acceptable, qui peut être choisi par l'homme du métier selon l'application envisagée.

Ce milieu peut comprendre une phase aqueuse et/ou une phase grasse. Il peut également être anhydre.
La phase aqueuse peut comprendre de l'eau et/ou une eau thermale et/ou une eau de source et/ou une eau minérale et/ou une eau florale.
Elle peut également comprendre un ou plusieurs solvants organiques cosmétiquement acceptables ou bien un mélange d'eau et d'un ou plusieurs solvants organiques cosmétiquement acceptables. Parmi ces solvants organiques, on peut citer :
- les alcools en C₁-C₄ tels que l'éthanol, l'isopropanol, le n-propanol ;
- les éthers tels que le diméthoxyéthane ;
- les cétones telles que l'acétone, la méthyléthylcétone ;
- les esters d'acide carboxylique inférieurs en C₁-C₃ tels que l'acétate de méthyle, l'acétate d'éthyle.

La phase grasse peut comprendre des huiles, volatiles ou non, des gommes et/ou des cires usuelles, d'origine animale, végétale, minérale ou synthétique, seules ou en mélanges, et notamment :
- des huiles de silicone, volatiles ou non, linéaires, ramifiées ou cycliques, éventuellement organomodifiées; des silicones phénylées; des résines et des gommes de silicone liquides à température ambiante;
- des huiles minérales telles que l'huile de paraffine et de vaseline,
- des huiles d'origine animale telles que le perhydrosqualène, la lanoline;
- des huiles d'origine végétale telles que les triglycérides liquides, par exemple les huiles de tournesol, de maïs, de soja, de jojoba, de courge, de pépins de raisin, de sésame, de noisette, d'abricot, de macadamia, d'avocat, d'amande douce, de ricin, les triglycérides des acides caprylique/caprique; l'huile d'olive, l'huile d'arachide, l'huile de colza, l'huile de coprah;
- des huiles de synthèse telles que l'huile de purcellin, les isoparaffines; les alcools gras; les esters d'acides gras;
- des huiles fluorées et perfluorées; des huiles de silicones fluorées;
- des cires choisies parmi les cires animales, fossiles, végétales, minérales ou de synthèse connues, telles que les cires de paraffine, les cires de polyéthylène, les cires de Camauba, de Candellila; les cires d'abeilles; la cire de lanoline, les cires d'insectes de Chine, la cire de riz, la cire d'Ouricurry, la cire d'Alfa, la cire de fibres de liège, la cire de canne à sucre, la cire du Japon, la cire de sumac, la cire de montan, les cires microcristallines, l'ozokérite, les cires obtenues par la synthèse de Fisher-Tropsch; les cires de silicone; leurs mélanges.

La composition peut en outre comprendre au moins un colorant hydrosoluble et/ou au moins un pigment, utilisés de manière usuelle dans le domaine de la cosmétique et du maquillage. Par pigments, il faut comprendre des particules blanches ou colorées, minérales ou organiques, insolubles dans le milieu, destinées à colorer et/ou opacifier la composition. Les pigments peuvent être présents dans la composition à raison de 0-20% en poids de la composition finale, et de préférence à raison de 1-5%. Ils peuvent être blancs ou colorés, minéraux et/ou organiques, de taille usuelle ou nanométrique. On peut citer, parmi les pigments et nanopigments minéraux, les oxydes de titane, de zirconium ou de cérium, ainsi que les oxydes de zinc, de fer ou de chrome, le bleu ferrique. Parmi les pigments organiques, on peut citer le noir de carbone, et les laques de baryum, strontium, calcium, aluminium. Parmi les colorants hydrosolubles, on peut citer les colorants usuels du domaine considéré tels que le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

Par ailleurs, la composition selon l'invention peut contenir des adjuvants couramment utilisés dans les compositions cosmétiques ou pharmaceutiques, notamment destinées à une application topique. En particulier, ces compositions peuvent comprendre :
- des actifs cosmétiques et/ou pharmaceutiques tels que les adoucissants, les antioxydants, les opacifiants, les émollients, les hydroxyacides, les agents anti-mousse, les hydratants, les vitamines, les parfums, les conservateurs, les séquestrants, des filtres UV, des céramides; des agents anti-radicaux libres; des agents amincissants; des bactéricides; des antipelliculaires; des complexants; des absorbeurs d'odeur; des actifs de soin tels que des antiacnéiques; des agents anti-chute des cheveux; des agents antifongiques ou antiseptiques; des antitranspirant, des anti-bactériens;
- des charges, des nacres, des laques; des épaississants, des gélifiants; des polymères notamment fixants ou conditionneurs; des agents propulseurs, des agents alcalinisants ou acidifiants; des plastifiants; des tensioactifs;
- des polymères hydrophiles additionnels, tels que les alcools polyvinyliques et leurs copolymères; les polysaccharides ou les polymères cellulosiques; les protéines naturelles ou les polypeptides synthétiques;
- des polymères hydrosolubles.

Bien entendu, l'homme du métier veillera à choisir ce ou ces éventuels adjuvants, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous différentes formes et en particulier sous forme d'émulsions huile-dans-eau ou eau-dans-huile; de dispersions aqueuses, huileuses ou en milieu solvant; de solutions aqueuses, huileuses ou en milieu solvant; sous forme fluide, épaissie ou gélifiée, semi-solide, pâte souple; sous forme solide telle que de stick ou bâton.

De préférence, elles se présentent sous forme semi-solide, pâteuse ou solide.

Les compositions selon l'invention trouvent une application dans un grand nombre de traitements cosmétique ou pharmaceutique de la peau, des cheveux, des cils, des sourcils, des ongles, des muqueuses, du cuir chevelu.

Elles trouvent une application toute particulière en tant que produit de maquillage du visage ou du corps, notamment en tant que rouge à lèvres, fond de teint, fard à joues ou fard à paupières, ou encore eye-liner et mascara.
On peut également envisager une application dans le domaine des compositions de soin de la peau du visage ou du corps, ou des cheveux, du cuir chevelu, des muqueuses ou semi-muqueuses; des compositions solaires ou autobronzantes; des compositions dermatologiques ou encore des compositions pharmaceutiques à appliquer sur la peau, les semi-muqueuses et/ou les muqueuses.
On peut aussi envisager une application comme composition d'hygiène corporelle par exemple sous forme de stick déodorant; ou comme composition capillaire par exemple comme stick de coiffage.

Plus préférentiellement, elles sont employées comme compositions de maquillage sans transfert, notamment comme composition de rouge à lèvres sans transfert, de préférence sous forme solide telle qu'un stick ou sous forme épaissie ou gélifiée; et/ou comme composition de fond de teint sans transfert sous forme de solide ou semi-solide, notamment de compact, ou sous forme de fluide éventuellement épaissi.

L'invention est illustrée plus en détail dans les exemples suivants.

### A/ Mesure de la dureté

La dureté du film est mesurée selon la norme ASTM D-43-66, ou la norme NF-T 30-016 (octobre 1981), à l'aide d'un pendule de Persoz.
Le film déposé sur le support doit avoir une épaisseur d'environ 300 microns avant séchage. Après séchage pendant 24 heures, à 30°C et sous une humidité relative de 50%, on obtient un film ayant une épaisseur d'environ 100 microns; on mesure alors sa dureté à 30°C et 50% d'humidité relative.

### B/ Mesure du module de Young (ou module d'élasticité)

Le module de Young (module d'élasticité) est mesuré selon la norme ASTM Standards, volume 06.01 D 2370-92 'Standard Test Method for Tensile Properties of Organic Coatings'.
Le film déposé sur le support doit avoir une épaisseur d'environ 300 microns avant séchage. Après séchage pendant 7 jours à 21°C et sous une humidité relative de 50%, on obtient un film ayant une épaisseur d'environ 100 microns.

Les échantillons mesurés ont une largeur de 5 mm et une épaisseur de 100 microns. La distance entre les mors est de 25 mm. La vitesse de traction est de 1000 mm par minute.

### Exemple 1 : Préparation de l'initiateur

L'initiateur préparé est le 5,11,17,23,29,35,41,47-octa-2-propionylbromide-49,50,51,52,53,54,55,56-octatertiobutyl-calix(8)arène (M = 2378 g).

Les réactifs utilisés sont les suivants :
- 4-tertiobutyl-calix(8)arène (M = 1298g) comportant 8 motifs phénols (Aldrich) 15 g
- 2-bromopropionylbromide de formule CH₃-CHBr-COBr 159,9 g
- triéthylamine 128 g
- tétrahydrofurane (THF) 1120 g

Dans un ballon muni d'agitation et d'un thermomètre, on ajoute le 4-tbutyl-calix(8)arène et le solvant THF; on laisse sous agitation pendant 10 minutes à température ambiante.
On ajoute ensuite la triéthylamine, ce qui prend environ 15 minutes.
On ajoute alors le 2-bromopropionylbromide préalablement dissous dans le THF, à une température de 5°C environ, ce qui prend 1h30 environ.
On laisse sous agitation durant 12 heures au moins, à 5°C, puis on laisse progressivement remonter la température jusqu'à température ambiante.
On concentre la solution obtenue par évaporation du THF. On précipite dans un mélange eau/glace, puis on extrait à l'éther éthylique et on sèche sur sulfate de magnésium.
On concentre la solution obtenue et on précipite dans un mélange méthanol/glace (90/10) dans un rapport composé/précipitant de 1/5.
On obtient 23 g de composé, se présentant sous forme de poudre, soit un rendement de 85%.

La caractérisation est effectuée par RMN/ GPC ou HPLC. Le composé obtenu présente des valeurs conformes à celles attendues.

### Exemple 2 : Préparation d'un polymère-étoile à 8 branches dont chaque branche est un copolymère bloc

### 1/ Première étape : préparation d'un polymère-étoile à 8 branches de polyacrylate de tertiobutyle

Les réactifs employés sont les suivants:
- monomère 1: acrylate de tertiobutyle (Tg = 50°C) 100 g
- monomère 2 : méthacrylate de lauryle (Tg = -20°C) 180 g
- initiateur (préparé selon l'exemple 1) (correspondant à 4.10⁻³ mole de RBr) 11,19 g
- CuBr (correspondant à 4.10⁻³ mol) 10,57 g
- Bipyridine (correspondant à 8.10⁻³ mol) 11,25 g

Les monomères sont préalablement distillés.
Dans un réacteur hermétique, flambé et comportant une arrivée d'azote, on mélange les réactifs sauf les monomères, puis on ajoute le monomère 1.
On chauffe, sous azote, à 120°C environ puis on laisse réagir à 120°C pendant 4 heures, en coupant l'arrivée d'azote.

### 2/Deuxième étape : formation du deuxième bloc à l'extrémité de chaque branche

On ajoute alors le monomère 2, à savoir 80 g de méthacrylate de lauryle. On laisse à nouveau réagir à 120°C, pendant 4 heures.

Après réaction, on laisse refroidir le mélange réactionnel; on obtient une solution visqueuse verte que l'on dissout dans le dichlorométhane. On passe la solution de polymère sur alumine neutre et on précipite la solution limpide obtenue dans un mélange méthanolleau (80/20) dans un rapport polymère/précipitant de 1/5.

On obtient 165 g de polymère se présentant sous forme de produit visqueux, soit un rendement de 90%.
Ce polymère est un copolymère bloc : calix(polyacrylate de tertiobutyle-bloc-polyméthacrylate de lauryle)8.

La caractérisation est effectuée par GPC : THF équivalent polystyrène linéaire, détection diffusion de lumière : 329 800 g/mol (masse théorique : 346 400 environ); indice de polydispersité : 2.
Le polymère obtenu présente des valeurs conformes à celles attendues.
Module de Young : 6 MPa
Dureté:15 s

### Exemple 3 : Rouge à lèvres

On prépare une solution à 25% dans l'isododécane du polymère préparé selon l'exemple 2.
On prépare ensuite une composition de rouge à lèvres comprenant :
- solution du polymère à 25% dans l'isododécane 5 g
- cires (Carnauba, polyéthylène) 20 g
- huiles (ricin, jojoba, coco hydrogénée) 35 g
- lanolate d'isopropyle 25 g
- charges/ pigments 15g

On obtient une composition qui peut être appliquée sur les lèvres, cosmétiquement satisfaisante et qui donne un film sans 'tack'.

### Exemple 4 : Fond de teint

On prépare une solution à 25% dans l'isododécane du polymère préparé selon l'exemple 2.
On prépare ensuite une composition de fond de teint comprenant :
- solution du polymère à 25% dans l'isododécane 82 g
- poudre de Nylon 8 g
- oxyde de titane 8 g
- oxydes de fer 2 g

On obtient un fond de teint qui peut être appliqué sur le cou et le visage. Le maquillage est naturel et ne présente pas d'inconfort. Il ne transfère pas et est résistant à l'eau.

## Revendications

1. Composition cosmétique de maquillage du visage ou du corps, comprenant, dans un milieu cosmétiquement acceptable comprenant au moins un constituant choisi parmi l'eau; un ou plusieurs solvants organiques cosmétiquement acceptables tels que les alcools en C₁-C₄, les éthers, les cétones, les esters d'acide carboxylique inférieurs en C₁-C₃; les huiles de silicone, minérales, d'origine animale ou végétale, de synthèse ou fluorées, volatiles ou non; les gommes; les cires animales, fossiles, végétales, minérales ou de synthèse; les colorants hydrosolubles; les pigments; les antioxydants, les opacifiants, les émollients, les hydroxyacides, les hydratants, les vitamines, les parfums, les conservateurs, les filtres UV, les céramides, les agents anti-radicaux libres; des charges, des nacres, des laques, des épaississants; des gélifiants; des plastifiants; des tensioactifs; des polymères hydrophiles ou hydrosolubles; un agent auxiliaire de filmification, tel qu'un agent plastifiant et/ou un agent de coalescence;
au moins un polymère de structure en "étoiles" susceptible d'être obtenu par polymérisation radicalaire par transfert d'atomes :
- d'un ou plusieurs monomères polymérisables radicalairement, choisis parmi :
(i) les esters acryliques ou méthacryliques obtenus à partir d'alcools aliphatiques, linéaires, ramifiés, cycliques et/ou d'alcools aromatiques, en C₁-C₂₀ tel que le (méth)acrylate de méthyle, le (méth)acrylate d'éthyle, le (méth)acrylate de propyle, le (méth)acrylate de butyle, le (méth)acrylate d'isobutyle, le (méth)acrylate de tertio-butyte ;
(ii) les (méth)acrylates d'hydroxyalkyle en C₁-C₄ tels que le (méth)acrylate de 2-hydroxyéthyle ou le (méth)acrylate de 2-hydroxypropyle;
(iii) les (méth)acrylates d'éthylèneglycol, de diéthylèneglycol, de polyéthylèneglycol, à extrémité hydroxyle ou éther;
(iv) les esters vinyliques, allyliques ou méthallyliques obtenus à partir d'alcools aliphatiques, linéaires ou ramifiés en C₁-C₁₀ ou cycliques en C₁-C₆ et/ou d'alcools aromatiques, en C₁-C₆, tels que l'acétate de vinyle, le propionate de vinyle, le benzoate de vinyle, le tertio-butyl benzoate de vinyle ;
(v) la N-vinylpyrrolidone; la vinylcaprolactame; les vinyl N-alkylpyrroles ayant 1 à 6 atomes de carbone; les vinyl-oxazoles; les vinyl-thiazoles; les vinylpyrrimidines; les vinylimidazoles; les vinyl cétones;
(vi) les amides (méth)acryliques obtenues à partir d'amines aliphatiques, linéaires, ramifiés, cycliques et/ou d'amines aromatiques, en C₁-C₂₀ telles que le tertiobutylacrylamide; les (méth)acrylamides tels que l'acrylamide, le méthacrylamide, les dialkyl(C₁-C₄) (méth)acrylamides ;
(vii) les oléfines tels que l'éthylène, le propylène, le styrène ou le styrène substitué;
(viii) les monomères acryliques ou vinyliques fluorés ou perfluorés, notamment les esters (méth)acryliques à motifs perfluoroalkyle;
(ix) les monomères comportant une fonction amine sous forme libre ou bien partiellement ou totalement neutralisée ou bien partiellement ou totalement quaternisée tels que le (méth)acrylate de diméthylaminoéthyle, le diméthylaminoéthyl méthacrylamide, la vinylamine, la vinylpyridine, le chlorure de diallyldiméthylammonium;
(x) les carboxybétaïnes ou les sulfobétaïnes obtenues par quaternisation partielle ou totale de monomères à insaturation éthylénique comportant une fonction amine par des sels de sodium d'acide carboxylique à halogénure mobile (chloroacétate de sodium par exemple) ou par des sulfones cycliques (propane sulfone);
(xi) les (méth)acrylates ou (méth)acrylamides siliconés, notamment les esters(méth)acryliques à motifs siloxane;
(xii) leurs mélanges;
en présence
- d'un initiateur ayant au moins deux atomes et/ou groupes radicalairement transférables par polymérisation,
- d'un composé comprenant un métal de transition, susceptible de participer à une étape de réduction avec l'initiateur et une chaîne polymérique "dormante", choisi parmi ceux de formule Mⁿ⁺X'ₙ, dans laquelle :
- M est choisi parmi Cu, Au, Ag, Hg, Ni, Pd, Pt, Rh, Co, Ir, Fe, Ru, Os, Re, Mn, Cr, Mo, W, V, Nb, Ta et Zn, et
- X' représente un halogène (brome ou chlore notamment), OH, (O)_{1/2}, un radical alcoxy ayant 1-6 atomes de carbone, (SO₄)_{1/2}, (PO₄)_{1/3}, (HPO₄)_{1/2}, (H₂PO₄), un radical triflate, hexafluorophosphate, methanesulfonate, arylsulfonate, SeR, CN, NC, SCN, CNS, OCN, CNO, N₃ et R'CO₂, dans lequel R représente un radical aryle ou alkyle, linéaire ou ramifié, ayant 1-20, de préférence 1-10 atomes de carbone, et R' représente H ou un radical alkyle, linéaire ou ramifié, ayant 1-6 atomes de carbone, ou un radical aryle, éventuellement substitué par un ou plusieurs atomes d'halogènes notamment de fluor et/ou de chlore;
- n est la charge du métal;
et
- d'un ligand choisi parmi :
(i) les composés comprenant au moins un atome d'azote (N), d'oxygène (O), de phosphore (P) et/ou de soufre (S), susceptibles de se coordonner par une liaison σ audit composé comprenant un métal de transition;
(ii) les composés comprenant au moins deux atomes de carbone susceptibles de se coordonner par une liaison π audit composé comprenant un métal de transition;
(iii) les composés comprenant au moins un atome de carbone susceptibles de se coordonner par une liaison σ audit composé comprenant un métal de transition, mais qui ne forment pas de liaison carbone-carbone avec le monomère lors de la polymérisation;
(iv) les composés susceptibles de se coordonner par une liaison 4 ou η audit composé comprenant un métal de transition;
ledit polymère comprenant un ou plusieurs monomères Mi dont l'homopolymère correspondant présente une Tg supérieure ou égale à 0°C, de préférence supérieure ou égale à 5°C, et encore mieux supérieure ou égale à 20°C; ce ou ces monomères Mi étant présents, dans le polymère final, en une quantité minimale de 40% en poids, de préférence en une quantité comprise entre 50 et 99% en poids, et encore mieux en une quantité de 60-90% en poids, par rapport au poids total de monomères;
et
ledit polymère comprenant par ailleurs un ou plusieurs monomères Mj dont l'homopolymère correspondant présente une Tg inférieure ou égale à 0°C, de préférence inférieure ou égale à -10°C, et encore mieux inférieure ou égale à -15°C; ce ou ces monomères Mj étant présents, dans le polymère final, en une quantité maximale de 60% en poids, de préférence en une quantité comprise entre 1 et 50% en poids, et encore mieux en une quantité de 10-40% en poids, par rapport au poids total de monomères.

2. Composition selon la revendication 1, dans laquelle les monomères sont choisis parmi :
- les esters (méth)acryliques obtenus à partir d'alcools aliphatiques, linéaires ou ramifiés, de préférence en C₁-C₂₀;
- les esters (méth)acryliques en C₁-C₂₀ à motifs perfluoroalkyle;
- les esters(méth)acryliques en C₁-C₂₀ à motifs siloxane;
- les amides (méth)acryliques obtenues à partir d'amines aliphatiques, linéaires, ramifiés, cycliques et/ou d'amines aromatiques, de préférence en C₁-C₂₀ telles que le tertiobutylacrylamide; les (méth)acrylamides tels que l'acrylamide, le méthacrylamide, les dialkyl(C₁-C₄) (méth)acrylamides ;
- les esters vinyliques, allyliques ou méthallyliques obtenus à partir d'alcools aliphatiques, linéaires ou ramifiés en C₁-C₁₀ ou cycliques en C₁-C₆ ;
- la vinylcaprolactame ;
- le styrène éventuellement substitué;
- leurs mélanges.

3. Composition selon l'une des revendications précédentes, dans laquelle l'initiateur est choisi parmi les composés de formule :
- R¹¹ₓ R¹²_{y} R¹³_{z} C-(RX)ₜ dans laquelle x, y et z représentent un entier allant de 0 à 4, t un entier allant de 1 à 4, et x+y+z= 4-t;
- R¹³ₓ C₆ -(RX)_{y} (cycle à 6 carbones, saturé) dans laquelle x représente un entier allant de 7 à 11, y représente un entier allant de 1 à 5, et x+y= 12 ;
- R¹³ₓ C₆ -(RX)_{y} (cycle à 6 carbones, insaturé) dans laquelle x représente un entier allant de 0 à 5, y représente un entier allant de 1 à 6, et x+y= 6;
- -[-(R¹¹)(R¹²)(R¹³)C-(RX)-]ₙ dans laquelle n est supérieur ou égal à 1; cyclique ou linéaire;
- -[-(R¹²)ₓ C₆ (RX)_{y}- R¹¹-]ₙ dans laquelle x représente un entier allant de 0 à 6, y représente un entier allant de 1 à 6, et n est supérieur ou égal à 1, avec x+y=4 ou 6; cyclique ou linéaire;
- -[-(R¹²)ₓ C₆ (RX)_{y}- R¹¹-]ₙ dans laquelle x représente un entier allant de 0 à 12, y représente un entier allant de 1 à 12, et n est supérieur ou égal à 1, avec x+y=10 ou 12; cyclique ou linéaire;
- -[OSi (R¹¹)ₓ(RX)_{y}] ₙ , cyclique ou linéaire, dans laquelle x et y représentent un entier allant de 0 à 2, et n est supérieur ou égal à 1, avec x+y=2;
- R¹¹N-X₂
- (R¹¹)ₓP(O)_{y}-X₃₋ₓ dans laquelle x et y représentent des entiers allant de 0 à 2, et x+y = 5;
- (R¹¹O)ₓP(O)_{y}-X₃₋ₓ dans laquelle x et y représentent des entiers allant de 0 à 2, et x+y = 5;
- -[(R¹¹)ₜN_{z}P(O)ₓ(O-RX)_{y}-]ₙ, cyclique ou linéaire, dans laquelle x représente un entier allant de 0 à 4, y représente un entier allant de 1 à 5, z représente un entier allant de 0 à 2 et t représente un entier allant de 0 à 3, et n est supérieur ou égal à 1;
dans lesquelles :
- R, R¹¹, R¹² et R¹³ représentent, indépendamment l'un de l'autre, un atome d'hydrogène ou d'halogène; un radical alkyle, linéaire ou ramifié, ayant 1-20, de préférence 1-10 et plus préférentiellement 1-6 atomes de carbone; un radical cycloalkyle ayant 3-8 atomes de carbone; un radical -C(=Y)R⁵, -C(=Y)NR⁶R⁷ ou -R⁸₃Si; -COCl, -OH, -CN, un radical alkényle ou alkynyle ayant 2-20, de préférence 2-6, atomes de carbone; un radical oxiranyle, glycidyle, alkylène ou alkénylène substitué avec un oxiranyle ou un glycidyle; un radical aryle, heterocyclyle, aralkyle, aralkenyle; un radical alkyle ayant 1-6 atomes de carbone dans lequel tout ou partie des atomes d'hydrogène sont substitués soit par des atomes d'halogènes tels que fluor, chlore ou brome, soit par un groupe alcoxy ayant 1-4 atomes de carbone ou par un radical aryle, heterocyclyle, -C(=Y)R⁵, -C(=Y)NR⁶R⁷, oxiranyle, glycidyle;
- X représente un atome d'halogène tel que Cl, Br, I, ou un radical -OR', -SR, -SeR, -OC(=O)R'. -OP(=O)R', -OP(=O)(OR')₂, -OP(=O)OR'-, -O-NR'₂, -S-C(=S)NR'₂, -CN, -NC, -SCN, -CNS, -OCN, -CNO et -N₃, dans lequel R' représente un radical alkyle ayant 1-20 atomes de carbone éventuellement substitué par un ou plusieurs atomes d'halogènes notamment de fluor et/ou de chlore; et R représente un radical aryle ou alkyle, linéaire ou ramifié, ayant 1-20, de préférence 1-10, atomes de carbone; le groupement -NR'₂ pouvant en outre représenter un groupement cyclique, les deux groupes R' étant joints de manière à former un hétérocycle à 5, 6 ou 7 membres;
- Y représente O, S ou NR⁸ (de préférence O),
- R⁵ représente un radical alkyle, alkylthio, alcoxy, linéaire ou ramifié, ayant 1-20 atomes de carbone; un radical OH; un radical OM' avec M' = métal alcalin; un radical aryloxy ou un radical heterocyclyloxy;
- R⁶ et R⁷ représentent, indépendamment l'un de l'autre, H ou un radical alkyle, linéaire ou ramifié, ayant 1-20 atomes de carbone; étant donné que R⁶ et R⁷ peuvent être joints pour former un groupe alkylène ayant 2-7, de préférence 2-5, atomes de carbone;
- R⁸ représente H; un radical alkyle, linéaire ou ramifié, ayant 1-20 atomes de carbone ou un radical aryle.

4. Composition selon l'une des revendications précédentes, dans laquelle l'initiateur est choisi parmi :
- l'octa-2-isobutyrylbromide-octatertiobutyl-calix(8)arène,
- l'octa-2-propionylbromide -octatertiobutyl-calix(8)arène, et
- l'hexakis α-bromométhylbenzène.

5. Composition selon l'une des revendications précédentes, dans laquelle le ligand est choisi parmi :
(i) les composés de formule :
R⁹-Z-(R¹⁴-Z)ₘ-R¹⁰ ou R²⁰R²¹C[C(=Y)R⁵]
dans lesquelles :
- R⁹ et R¹⁰ sont, indépendamment l'un de l'autre, un atome d'hydrogène; un radical alkyle, linéaire ou ramifié, ayant 1-20, de préférence 1-10 atomes de carbone; un radical aryle; un radical hétérocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸, R⁵ à R⁸ et Y ayant les définitions données en revendication 3; étant donné que R⁹ et R¹⁰ peuvent être joints de manière à former un cycle, saturé ou insaturé;
- R¹⁴ représente, indépendamment les uns des autres, un groupe divalent choisi parmi les alcanediyles ayant 2-4 atomes de carbone; les alkénylènes ayant 2-4 atomes de carbone; les cycloalcanediyles ayant 3-8 atomes de carbone; les cycloalkènediyles ayant 3-8 atomes de carbone; les arènediyles et les hétérocyclylènes;
- Z représente O, S, NR¹⁵ ou PR¹⁵ avec R¹⁵ représentant H; un radical alkyle, linéaire ou ramifié, ayant 1-20 atomes de carbone; un radical aryle; un radical heterocyclyle; un radical alkyle ayant 1-6 atomes de carbone substitué avec un radical alcoxy ayant 1-6 atomes de carbone ou un radical dialkylamino ayant 1-4 atomes de carbone ou un radical -C(=Y)R⁵ ou -C(=Y)NR⁶R⁷ et/ou YC(=Y)R⁸, R⁵ à R⁸ et Y ayant les définitions données dans la revendication 3;
- m est compris entre 0 et 6;
- R²⁰ et R²¹ sont, indépendamment l'un de l'autre, un atome d'hydrogène; un atome d'halogène; un radical alkyle, linéaire ou ramifié, ayant 1-20, de préférence 1-10 atomes de carbone; un radical aryle; un radical hétérocyclyle; étant donné que R²⁰ et R²¹ peuvent être joints de manière à former un cycle, saturé ou insaturé; étant donné que chaque radical peut en outre être substitué avec un radical alkyle ayant 1-6 atomes de carbone, un radical alcoxy ayant 1-6 atomes de carbone ou un radical aryle;
(ii) le monoxyde de carbone; les porphyrines et les porphycènes, éventuellement substitués; l'éthylènediamine et le propylènediamine, éventuellement substitués; les multiamines avec amines tertiaires telles que le pentaméthyldiéthylènetriamine; les aminoalcools tels que l'aminoéthanol et l'aminopropanol, éventuellement substitués; les glycols tels que l'éthylèneglycol ou le propylèneglycol, éventuellement substitués; les arènes tels que le benzène, éventuellement substitués; le cyclopentadiène, éventuellement substitué; les pyridines et bipyridines, éventuellement substituées; l'acétonitrile; la 1,10-phénanthroline; les cryptands et les éthers-couronnes; la spartéine.

6. Composition selon l'une des revendications précédentes, dans laquelle le polymère de structure "en étoiles" permet l'obtention d'un film :
- ayant un module d'élasticité (module de Young) inférieur à environ 200 MPa, de préférence compris entre 2 et 100 MPa, préférentiellement compris entre 5 et 80 MPa; et/ou
- ayant une dureté inférieure à 110, de préférence comprise entre 1 et 70 et encore mieux comprise entre 5 et 55.

7. Composition selon l'une des revendications précédentes, dans laquelle le polymère de structure 'en étoiles' est présent en une quantité comprise entre 1-50% en poids de matière sèche, par rapport au poids total de la composition, de préférence entre 1-40% en poids et préférentiellement entre 5-35% en poids.

8. Composition selon l'une des revendications précédentes, dans laquelle le polymère de structure 'en étoiles' est présent dans le milieu sous forme dissoute ou en dispersion, dans une phase aqueuse, organique, hydroorganique et/ou grasse.

9. Composition selon l'une des revendications précédentes, se présentant sous forme d'émulsions huile-dans-eau ou eau-dans-huile; de dispersions aqueuses, huileuses ou en milieu solvant; de solutions aqueuses, huileuses ou en milieu solvant; sous forme fluide, épaissie ou gélifiée, semi-solide, pâte souple; sous forme solide telle que de stick ou bâton.

10. Composition selon l'une des revendications précédentes, se présentant sous la forme d'un rouge à lèvres, fond de teint, fard à joues, fard à paupières, eye-liner ou d'un mascara.

11. Composition selon l'une des revendications précédentes, se présentant sous la forme d'une composition de maquillage sans transfert, notamment comme composition de rouge à lèvres sans transfert et/ou comme composition de fond de teint sans transfert.

12. Procédé de traitement cosmétique des matières kératiniques telles que la peau, les cils, les sourcils, les ongles, les lèvres, **caractérisé en ce qu'**il consiste à appliquer sur ces dernières une composition cosmétique selon l'une des revendications 1 à 11.

13. Utilisation d'au moins un polymère tel que défini dans la revendication 1, dans une composition cosmétique pour diminuer, voire supprimer, le transfert du film de composition déposé.

## Patentansprüche

1. Kosmetische Zusammensetzung zum Schminken des Gesichts oder des Körpers, die in einem kosmetisch akzeptablen Medium mindestens einen Bestandteil, der unter den folgenden Verbindungen ausgewählt ist: Wasser; einem oder mehreren kosmetisch akzeptablen organischen Lösungsmitteln, wie C₁₋₄-Alkoholen, Ethern, Ketonen, Estern von niederen Carbonsäuren mit 1 bis 3 Kohlenstoffatomen; Siliconölen, Mineralölen, Ölen tierischer Herkunft, Ölen pflanzlicher Herkunft, synthetischen Ölen oder fluorierten Ölen, die flüchtig oder nicht flüchtig sind; Gummis; tierischen, fossilen, pflanzlichen, mineralischen oder synthetischen Wachsen; wasserlöslichen Farbstoffen; Pigmenten; Antioxidantien, Trübungsmitteln, Emollientien, Hydroxysäuren, Hydratisierungsmitteln, Vitaminen, Parfums, Konservierungsrnitteln, UV-Filtern, Ceramiden, Radikalfängern für freie Radikale; Füllstoffen, Perlglanzpigmenten, Lacken, Verdickungsmitteln; Gelbildnern; Weichmachern; grenzflächenaktiven Stoffen; hydrophilen oder wasserlöslichen Polymeren; Hilfsmitteln für die Filmbildung, wie Weichmachern und/oder Koaleszenzmitteln;
und mindestens ein Polymer mit "sternförmiger" Struktur enthält, das erhältlich ist durch radikalische Atomtransferpolymerisation:
- eines oder mehrerer radikalisch polymerisierbarer Monomere, die ausgewählt sind unter:
(i) den Acrylestern oder Methacrylestern, die ausgehend von aliphatischen, geradkettigen, verzweigten, cyclischen und/oder aromatischen Alkoholen mit 1 bis 20 Kohlenstoffatomen hergestellt werden, wie beispielsweise Methyl(meth)acrylat, Ethyl(meth)acrylat, Propyl(meth)acrylat, Butyl(meth)acrylat, Isobutyl(meth)acrylat und t-Butyl(meth)acrylat;
(ii) C₁₋₄-Hydroxyalkyl(meth)acrylaten, wie 2-Hydroxyethyl(meth)acrylat oder 2-Hydroxypropyl(meth)acrylat;
(iii) Ethylenglykol(meth)acrylaten, Diethylenglykol(meth)-acrylaten und Polyethylenglykol(meth)acrylaten mit endständigen Hydroxy- oder Ethergruppen;
(iv) Vinylestern, Allylestern oder Methallylestem, die ausgehend von aliphatischen, geradkettigen oder verzweigten C₁₋₁₀-Alkoholen oder cyclischen C₁₋₆-Alkohlen und/oder aromatischen Alkoholen mit 1 bis 6 Kohlenstoffatomen hergestellt werden, beispielsweise Vinylacetat, Vinylpropionat, Vinylbenzoat und Vinyl-*t*-butylbenzoat;
(v) N-Vinylpyrrolidon; Vinylcaprolactam; Vinyl-N-alkylpyrrolen mit 1 bis 6 Kohlenstoffatomen; Vinyloxazolen, Vinylthiazolen; Vinylpyrimidinen; Vinylimidazolen; und Vinylketonen;
(vi) (Meth)acrylamiden, die ausgehend von aliphatischen, geradkettigen, verzweigten, cyclischen und/oder aromatischen Aminen mit 1 bis 20 Kohlenstoffatomen hergestellt werden, wie *t*-Butylacrylamid; (Meth)acrylamiden, wie Acrylamid, Methacrylamid und Di-C₁₋₄alkyl(meth)acrylamiden;
(vii) Olefinen, wie Ethylen, Propylen, Styrol oder substituiertern Styrol;
(viii) fluorierten oder perfluorierten Acrylmonomeren oder Vinylmonomeren, insbesondere (Meth)acrylestern mit Perfluoralkylgruppen;
(ix) Monomeren, die eine Aminogruppe in freier Form oder ganz oder teilweise neutralisiert oder ganz oder teilweise quaternisiert enthalten, wie Dimethylaminoethyl(meth)-acrylat, Dimethylaminoethylmethacrylamid, Vinylamin, Vinylpyridin und Diallyldimethylammoniumchlorid;
(x) Carboxybetainen oder Sulfobetainen, die durch partielle oder vollständige Quaternisierung von Monomeren mit ethylenischer Doppelbindung, die eine Aminogruppe enthalten, durch Natriumsalze von Carbonsäuren mit mobilem Halogenid (beispielsweise Natriumchloracetat) oder durch cyclische Sulfone (Propansulfon) hergestellt werden;
(xi) siliconhaltigen (Meth)acrylaten oder (Meth)acrylamiden, insbesondere den (Meth)acrylestern mit Siloxangruppen; und
(xii) deren Gemischen.
in Gegenwart
- eines Initiators, der mindestens zwei durch Polymerisation radikalisch transferierbare Atome und/oder Gruppen aufweist,
- einer Verbindung, die ein Übergangsmetall enthält, das an einem Reduktionssehritt mit dem Initiator und einer "schlafenden" Polymerkette teilnehmen kann, und die unter den Verbindungen der Formel Mⁿ⁺X'ₙ ausgewählt ist, worin:
- M unter Cu, Au, Ag, Hg, Ni, Pd, Pt, Rh, Co, Ir, Fe, Ru, Os, Re, Mn, Cr, Mo, W, V, Nb, Ta und Zn ausgewählt ist,
- X' ein Halogen (insbesondere Brom oder Chlor), OH, (O)_{1/2}, eine Alkoxygruppe mit 1 bis 6 Kohlenstoffatomen, (SO₄)_{1/2}, (PO₄)_{1/3}, (HPO₄)_{1/2}, (H₂PO₄), eine Triflatgruppe, Hexafluorphosphat, Methansulfonat, Arylsulfonat, SeR, CN, NC, SCN, CNS, OCN, CNO, N₃ et R'CO₂ bedeuten kann,
wobei R eine geradkettige oder verzweigte Aryl- oder Alkylgruppe mit 1 bis 20 und vorzugsweise 1 bis 10 Kohlenstoffatomen ist und R' H oder eine geradkettige oder verzweigte Alkylgruppe mit 1 bis 6 Kohlenstoffatomen oder eine Arylgruppe bedeutet, die ggf. mit einem oder mehreren Halogenatomen und insbesondere Fluor und/oder Chlor substituiert ist; und
- n die Ladung des Metalls bedeutet; und
- eines Liganden, der ausgewählt ist unter:
(i) den Verbindungen, die mindestens ein Stickstoffatom (N), Sauerstoffatom (O), Phosphoratom (P) und/oder Schwefelatom (S) enthalten und die über eine σ-Bindung mit der Verbindung, die ein Übergangsmetall enthält, eine Koordinationsverbindung bilden können;
(ii). Verbindungen, die mindestens zwei Kohlenstoffatome aufweisen und die über eine π-Bindung mit der Verbindung, die ein Übergangsmetall enthält, eine Koordinationsverbindung bilden können;
(iii) Verbindungen, die mindestens ein Kohlenstoffatom enthalten und die über eine σ-Bindung mit der Verbindung, die ein Übergangsmetall enthält, eine Koordinationsverbindung bilden können, die jedoch mit dem Monomer bei der Polymerisation keine Kohlenstoff-Kohlenstoff-Bindung ausbilden; und
(iv) Verbindungen, die über eine µ- oder η-Bindung mit der Verbindung, die ein Übergangsmetall enthält, eine Koordinationsverbindung bilden können;
wobei das Polymer ein oder mehrere Monomere Mi enthält, deren korrespondierendes Homopolymer eine Tg von 0°C oder darüber, vorzugsweise mindestens 5°C und noch besser mindestens 20°C aufweist; wobei das Monomer Mi oder die Monomere Mi in dem fertigen Polymer in einer Menge von mindestens 40 Gew.-%, vorzugsweise 50-99 Gew.-% und noch besser 60-90 Gew.-%, bezogen auf das Gesamtgewicht der Monomere, vorliegen; und
wobei das Polymer im Übrigen ein oder mehrere Monomere Mj enthält; deren korrespondierendes Homopolymer eine Tg von 0°C oder darunter, vorzugsweise höchstens -10°C und noch besser höchstens -15°C aufweist; wobei das Monomer Mj oder die Monomere Mj in dem fertigen Polymer in einer Menge von höchstens 60 Gew.-%, vorzugsweise in einer Menge von 1-50 Gew.-% und noch besser in einer Menge von 10-40 Gew.-%, bezogen auf das Gesamtgewicht der Monomere, vorliegt.

2. Zusammensetzung nach Anspruch 1, wobei die Monomere ausgewählt sind unter:
- (Meth)acrylestern, die ausgehend von aliphatischen, geradkettigen oder verzweigten Alkoholen mit vorzugsweise mit 1 bis 20 Kohlenstoffatomen hergestellt werden;
- (Meth)acrylestern mit 1 bis 20 Kohlenstoffatomen, die Perfluoralkylgruppen enthalten;
- (Meth)acrylestern mit 1 bis 20 Kohlenstoffatomen, die Siloxaneinheiten enthalten;
- (Meth)acrylamiden, die ausgehend von aliphatischen, geradkettigen, verzweigten, cyclischen und/oder aromatischen Aminen, vorzugsweise mit 1 bis 20 Kohlenstoffatomen, hergestellt werden, wie *t*-Butylacrylamid; (Meth)acrylamiden, wie Acrylamid, Methacrylamid, Di-C₁₋₄-alkyl(meth)acrylamiden;
- Vinylestern, Allylestern oder Methallylestern, die ausgehend von aliphatischen, geradkettigen oder verzweigten C₁₋₁₀-Alkoholen oder cyclischen C₁₋₆-Alkohlen hergestellt werden;
- Vinylcaprolactam;
- ggf. substituiertem Styrol; und
- deren Gemischen.

3. Zusammensetzung nach einem der vorhergehenden Ansprüche, wobei der Initiator unter den Verbindungen der folgenden Formeln ausgewählt ist:
- R¹¹ₓ R¹²_{y} R¹³_{z} C-(RX)ₜ, in der x, y und z 0 oder eine ganze Zahl von 1 bis 4 bedeuten, t eine ganze Zahl von 1 bis 4 ist und x+y+z=4-t;
- R¹³ₓ C₆-(RX)_{y} (gesättigter Ring mit 6 Kohlenstoffatomen), wobei x eine ganze Zahl von 7 bis 11 bedeutet, y eine ganze Zahl von 1 bis 5 ist und x+y=12;
- R¹³ₓ C₆-(RX)_{y} (ungesättigter Ring mit 6 Kohlenstoffatomen),
wobei x 0 oder eine ganze Zahl von 1 bis 5 bedeutet, y eine ganze Zahl von 1 bis 6 ist und x+y=6;
- [-R¹¹)(R¹²)(R¹³)C-(RX)-]ₙ, wobei n 1 bedeutet oder darüber liegt und die Verbindung cyclisch oder geradkettig vorliegt;
- [-(R¹²)ₓC₆(RX)_{y}-R¹¹-]ₙ, wobei x 0 oder eine ganze Zahl von 1 bis 6 bedeutet, y eine ganze Zahl von 1 bis 6 ist, und n 1 bedeutet oder darüber liegt, mit x+y=4 oder 6; wobei die Verbindung cyclisch oder geradkettig ist;
- [-(R¹²)ₓC₆(RX)_{y}-R¹¹-]ₙ, wobei x 0 oder eine ganze Zahl von 1 bis 12 bedeutet, y eine ganze Zahl von 1 bis 12 ist, und n 1 bedeutet oder darüber liegt, mit x+y=10 oder 12; wobei die Verbindung cyclisch oder geradkettig ist;
- -[OSi(R¹¹)ₓ(RX)_{y}]ₙ, wobei die Verbindung cyclisch oder geradkettig vorliegt und wobei x und y 0 oder eine ganze Zahl von 1 bis 2 bedeuten und n 1 ist oder darüber liegt, mit x+y=2;
- R¹¹N-X₂
- (R¹¹)ₓP(O)_{y}-X₃₋ₓ, wobei x und y 0 oder ganze Zahlen von 1 bis 2 bedeuten und x+y=5;
- (R¹¹O)ₓP(O)_{y}-X₃₋ₓ, wobei x und y 0 oder ganze Zahlen von 1 bis 2 bedeuten, und x+y=5;
- -[(R¹¹)ₜN_{z}P(O)ₓ(O-RX)_{y}-]ₙ, wobei die Verbindung cyclisch oder geradkettig vorliegt und wobei x 0 oder eine ganze Zahl von 1 bis 4 bedeutet, y eine ganze Zahl von 1 bis 5 ist, z 0 oder eine ganze Zahl von 1 bis 2 bedeutet, t 0 oder eine ganze Zahl von 1 bis 3 ist und n 1 bedeutet oder darüber liegt;
worin bedeuten:
- die Gruppen R, R¹¹, R¹² und R¹³ unabhängig voneinander ein Wasserstoffatom; ein Halogenatom; eine geradkettige oder verzweigte Alkylgruppe mit 1 bis 20 Kohlenstoffatomen, vorzugsweise 1 bis 10 Kohlenstoffatomen und besonders bevorzugt 1 bis 6 Kohlenstoffatomen; eine Cycloalkylgruppe mit 3 bis 8 Kohlenstoffatomen; eine Gruppe -C(=Y)R⁵, -C(=Y)NR⁶R⁷ oder -R⁸₃Si; -COCl, -OH, -CN, eine Alkenyl- oder Alkinylgruppe mit 2 bis 20 Kohlenstoffatomen und vorzugsweise 2 bis 6 Kohlenstoffatomen; eine Oxiranylgruppe, eine Glycidylgruppe, eine Alkylen- oder Alkenylengruppe, die mit einer Oxiranylgruppe oder einer Glycidylgruppe substituiert ist; Aryl, Heterocyclyl, Aralkyl, Aralkenyl; eine Alkylgruppe mit 1 bis 6 Kohlenstoffatomen, in der die Wasserstoffatome ganz oder teilweise entweder durch Halogenatome, wie Fluor, Chlor oder Brom, oder durch eine Alkoxygruppe mit 1 bis 4 Kohlenstoffatomen oder durch eine Arylgruppe, eine Heterocyclylgruppe, -C(=Y)R⁵, -C(=Y)NR⁶R⁷, Oxiranyl oder Glycidyl ersetzt sind;
- X ein Halogenatom, wie Cl, Br oder I, oder eine Gruppe -OR', -SR, -SeR, -OC(=O)R', OP(=O)R', -OP(=O)(OR')₂, -OP(=O)OR', -O-NR'₂, -S-C(=S)NR'₂, -CN, -NC, -SCN, -CNS, -OCN, -CNO und -N₃, wobei R' eine Alkylgruppe mit 1 bis 20 Kohlenstoffatomen bedeutet, die ggf. mit einem oder mehreren Halogenen, insbesondere Fluor und/oder Chlor substituiert ist; und R eine geradkettige oder verzweigte Aryl- oder Alkylgruppe mit 1 bis 20 Kohlenstoffatomen und vorzugsweise 1 bis 10 Kohlenstoffatomen bedeutet; wobei die Gruppe -NR'₂ ferner eine cyclische Gruppe bedeuten kann, in der die beiden Gruppen R' so verbunden sind, dass sie einen 5-, 6- oder 7-gliedrigen Heterocyclus bilden können;
- Y O, S oder NR⁸ (vorzugsweise O),
- R⁵ eine geradkettige oder verzweigte Alkyl-, Alkylthio- oder Alkoxygruppe mit 1 bis 20 Kohlenstoffatomen; OH; OM' mit M' = Alkalimetall; eine Aryloxy- oder eine Heterocyclyloxygruppe;
- R⁶ und R⁷ unabhängig voneinander H oder eine geradkettige oder verzweigte Alkylgruppe mit 1 bis 20 Kohlenstoffatomen,
wobei R⁶ und R⁷ gemeinsani eine Alkylengruppe mit 2 bis 7 und vorzugsweise 2 bis 5 Kohlenstoffatomen bilden können;
- R⁸ H; eine geradkettige oder verzweigte Alkylgruppe mit 1 bis 20 Kohlenstoffatomen oder eine Arylgruppe.

4. Zusammensetzung nach einem der vorhergehenden Ansprüche, wobei der Initiator ausgewählt ist unter:
- Octa-2-isobutyrylbromid-octa-*t*-butyl-calix(8)aren,
- Octa-2-propionylbromid-octa-t-butyl-calix(8)aren, und
- Hexakis-α-brommethylbenzol.

5. Zusammensetzung nach einem der vorhergehenden Ansprüche, wobei der Ligand ausgewählt ist unter:
(i) Verbindungen der Formel: R⁹-Z-(R¹⁴-Z)ₘ-R¹⁰ oder R²⁰R²¹C[C(=Y)R⁵], worin bedeuten:
- R⁹ und R¹⁰ unabhängig voneinander Wasserstoff; eine geradkettige oder verzweigte Alkylgruppe mit 1 bis 20 und vorzugsweise 1 bis 10 Kohlenstoffatomen; eine Arylgruppe; eine Heterocyclylgruppe; eine Alkylgruppe mit 1 bis 6 Kohlenstoffatomen, die mit einer Alkoxygruppe mit 1 bis 6 Kohlenstoffatomen oder einer Dialkylaminogruppe mit 1 bis 4 Kohlenstoffatomen oder einer Gruppe -C(=Y)R⁵ oder -C(=Y)NR⁶R⁷ und/oder YC(-Y)R⁸ (wobei die Definitionen für R⁵ bis R⁸ und Y in Anspruch 3 angegeben sind) substituiert ist; mit der Maßgabe, dass R⁹ und R¹⁰ gemeinsam einen gesättigten oder ungesättigten Ring bilden können;
- die Gruppen R¹⁴ unabhängig voneinander eine zweiwertige Gruppe, die unter den Alkandiylgruppen mit 2 bis 4 Kohlenstoffatomen; den Alkenylengruppen mit 2 bis 4 Kohlenstoffatomen, den Cycloalkandiylgruppen mit 3 bis 8 Kohlenstoffatomen; den Cycloalkendiylgruppen mit 3 bis 8 Kohlenstoffatomen, den Arendiylgruppen und den Heterocyclylgruppen ausgewählt sind;
- Z O, S, NR¹⁵ oder PR¹⁵ mit R¹⁵=H, eine geradkettige oder verzweigte Alkylgruppe mit 1 bis 20 Kohlenstoffatomen, eine Arylgruppe, eine Heterocyclylgruppe; eine Alkylgruppe mit 1 bis 6 Kohlenstoffatomen, die mit einer Alkoxygruppe mit 1 bis 6 Kohlenstoffatomen oder einer Dialkylaminogruppe mit 1 bis 4 Kohlenstoffatomen oder einer Gruppe -C(=Y)R⁵ oder -C(=Y)NR⁶R⁷ und/oder YC(=Y)R⁸ (wobei die Definitionen für die Gruppen R⁵ bis R⁸ und Y in Anspruch3 angegeben sind) substituiert ist;
- m eine Zahl im Bereich von 0 bis 6;
- R²⁰ und R²¹ unabhängig voneinander Wasserstoff; ein Halogenatom; eine geradkettige oder verzweigte Alkylgruppe mit 1 bis 20 und vorzugsweise 1 bis 10 Kohlenstoffatomen; eine Arylgruppe; eine Heterocyclylgruppe; mit der Maßnahme, dass R²⁰ und R²¹ auch gemeinsam einen gesättigten oder ungesättigten Ring bilden können und mit der Maßgabe, dass jede Gruppe ferner mit einer C₁₋₆-Alkylgruppe, einer C₁₋₆-Alkoxygruppe oder einer Arylgruppe substituiert sein kann; und
(ii) Kohlenmonoxid; Porphyrinen und Porphycenen, die ggf. substituiert sind; Ethylendiamin und Propylendiamin, die ggf. substituiert sind; Multiaminen mit tertiären Aminen, wie Pentamethyldiethylentriamin; Aminoalkoholen wie Aminoethanol und Aminopropanol, die ggf. substituiert sind; Glykolen, wie Ethylenglykol oder Propylenglykol, die ggf. substituiert sind; Arenen wie Benzol, die ggf. substituiert sind; Cyclopentadien, das ggf. substituiert ist; Pyridinen und Bipyridinen, die ggf. substituiert sind; Acetonitril; 1,10-Phenanthrolin; Kryptanden und Kronenether; und Spartein.

6. Zusammensetzung nach einem der vorhergehenden Ansprüche, wobei mit dem Polymer mit "sternförmiger" Struktur ein Film mit den folgenden Eigenschaften erzeugt werden kann:
· einem Elastizitätsmodul (Young-Modul) unter etwa 200 MPa, vorzugsweise im Bereich von 2 bis 100 MPa und noch bevorzugter 5 bis 80 MPa; und/oder
· einer Härte unter 110, vorzugsweise im Bereich von 1 bis 70 und noch besser 5 bis 55.

7. Zusammensetzung nach einem der vorhergehenden Ansprüche, wobei das Polymer mit "sternförmiger" Struktur in einer Menge von 1 bis 50 Gew.-% Trockensubstanz, bezogen auf das Gesamtgewicht der Zusammensetzung, vorzugsweise 1 bis 40 Gew.-% und noch bevorzugter 5 bis 35 Gew.-% vorliegt.

8. Zusammensetzung nach einem der vorhergehenden Ansprüche, wobei das Polymer mit sternförmiger" Struktur in dem Medium in gelöster Form oder in Form einer Dispersion in einer wässrigen Phase, organischen Phase, wässrig-organischen Phase und/oder Fettphase vorliegt.

9. Zusammensetzung nach einem der vorhergehenden Ansprüche, die als Öl-in-Wasser-Emulsion, Wasser-in-öl-Emulsion; wässrige Dispersion, ölige Dispersion oder Dispersion in einem Lösungsmittelmedium; wässrige Lösung, ölige Lösung oder Lösung in einem Lösungsmittelmedium; in fluider, verdickter oder gelierter, halbfester Form, als weiche Paste; oder in fester Form, z.B. als Stick oder Stift vorliegt.

10. Zusammensetzung nach einem der vorhergehenden Ansprüche, die als Lippenstift, Make-up, Wangenrouge, Lidschatten, Eyeliner oder Mascara vorliegt.

11. Zusammensetzung nach einem der vorhergehenden Ansprüche, die als Zusammensetzung zum Schminken ohne Transfer vorliegt, insbesondere als Lippenstift ohne Transfer und/oder als Make-up ohne Transfer.

12. Verfahren zur kosmetischen Behandlung von Keratinsubstanzen, beispielsweise zur Behandlung der Haut, der Wimpern, der Augenbrauen, der Nägel und der Lippen, das **dadurch gekennzeichnet ist, dass** es darin besteht, auf diese eine kosmetische Zusammensetzung nach einem der Ansprüche 1 bis 11 aufzubringen.

13. Verwendung mindestens eines in Anspruch 1 definierten Polymers in einer kosmetischen Zusammensetzung, um den Transfer eines abgeschiedenen Films der Zusammensetzung zu vermindern oder zu verhindern.

## Claims

1. Cosmetic composition for making up the face or the body, comprising, in a cosmetically acceptable medium comprising at least one constituent chosen from water; one or more cosmetically acceptable organic solvents, such as alcohols comprising 1 to 4 carbon atoms, ethers, ketones or C₁-C₃ lower carboxylic acid esters; silicone oils, mineral oils, oils of animal or vegetable origin, synthetic oils or fluorinated oils, which may or may not be volatile; gums; animal, fossil, vegetable, mineral or synthetic waxes; water-soluble dyes; pigments; antioxidants, opacifying agents, emollients, hydroxy acids, moisturizing agents, vitamins, fragrances, preservatives, UV screening agents, ceramides or agents for combating free radicals; fillers, pearlescence agents, lakes or thickeners; gelling agents; plasticizers; surfactants; hydrophilic or water-soluble polymers; or an additional agent which is able to form a film, such as a plasticizing agent and/or a coalescence agent;
at least one polymer with a "star" structure capable of being obtained by atom transfer radical polymerization:
- of one or more radically polymerizable monomers, chosen from:.
(i) acrylic or methacrylic esters obtained from linear, branched or cyclic aliphatic alcohols and/or from aromatic alcohols comprising 1 to 20 carbon atoms, such as methyl (meth)acrylate, ethyl (meth)acrylate, propyl (meth)acrylate, butyl (meth)acrylate, isobutyl (meth)acrylate or tert-butyl (meth)acrylate;
(ii) C₁-C₄ hydroxyalkyl (meth)acrylates, such as 2-hydroxyethyl (meth)acrylate, or 2-hydroxypropyl (meth)acrylate;
(iii) ethylene glycol, diethylene glycol or polyethylene glycol (meth)acrylates with a hydroxyl or ether end;
(iv) vinyl, allyl or methallyl esters obtained from linear or branched C₁-C₁₀ or cyclic C₁-C₆ aliphatic alcohols and/or from C₁-C₆ aromatic alcohols, such as vinyl acetate, vinyl propionate, vinyl benzoate or vinyl tert-butylbenzoate;
(v) N-vinylpyrrolidone; vinylcaprolactam; vinyl-N-alkylpyrroles having 1 to 6 carbon atoms; vinyl-oxazoles; vinylthiazoles; vinylpyrimidines; vinylimidazoles; or vinyl ketones;
(vi) (meth)acrylamides obtained from linear, branched or cyclic aliphatic amines and/or from aromatic amines comprising 1 to 20 carbon atoms, such as tert-butylacrylamide; or (meth)acrylamides, such as acrylamide, methacrylamide or di(C₁-C₄)alkyl(meth)-acrylamides;
(vii) olefins, such as ethylene, propylene, styrene or substituted styrene;
(viii) fluorinated or perfluorinated acrylic or vinyl monomers, in particular (meth)acrylic esters with perfluoroalkyl units;
(ix) monomers comprising an amine functional group in the free or else partially or completely neutralized or else partially or completely quaternized form, such as dimethylaminoethyl (meth)acrylate, dimethylaminoethyl-methacrylamide, vinyl amine, vinylpyridine or diallyldimethylammonium chloride;
(x) carboxybetaines or sulphobetaines obtained by partial or complete quaternization of monomers comprising ethylenic unsaturation comprising an amine functional group by sodium salts of carboxylic acids comprising a mobile halide (sodium chloroacetate, for example) or by cyclic sulphones (propane sulphone) ;
(xi) silicone-comprising (meth)acrylates or (meth)acrylamides, in particular (meth)acrylic esters comprising siloxane units;
(xii) their mixtures;
in the presence
- of an initiator having at least two atoms and/or groups radically transferable by polymerization,
- of a compound comprising a transition metal, capable of participating in a reduction stage with the initiator and a "dormant" polymer chain, chosen from those of formula Mⁿ⁺X'ₙ, in which:
- M is chosen from Cu, Au, Ag, Hg, Ni, Pd, Pt, Rh, Co, Ir, Fe, Ru, Os, Re, Mn, Cr, Mo, W, V, Nb, Ta and Zn, and
- X' represents a halogen (in particular bromine or chlorine), OH, (O)_{1/2,} an alkoxy radical having 1-6 carbon atoms, (SO₄)_{1/2}, (PO₄)_{1/3}, (HPO₄)_{1/2}, (H₂PO₄) and a triflate, hexafluorophosphate, methanesulphonate, arylsulphonate, SeR, CN, NC, SCN, CNS, OCN, CNO, N₃ and R'CO₂ radical, in which R represents an aryl or linear or branched alkyl radical having 1-20, preferably 1-10, carbon atoms and R' represents H or a linear or branched alkyl radical having 1-6 carbon atoms or an aryl radical which is optionally substituted by one or more halogen atoms, in particular fluorine and/or chlorine atoms;
- n is the charge on the metal;
and
- of a ligand chosen from:
(i) compounds comprising at least one nitrogen (N), oxygen (O), phosphorus (P) and/or sulphur (S) atom which are capable of coordinating via a σ bond to the said compound comprising a transition metal;
(ii) compounds comprising at least two carbon atoms capable of coordinating via a π bond to the said compound comprising a transition metal;
(iii) compounds comprising at least one carbon atom capable of coordinating via a σ bond to the said compound comprising a transition metal but which do not form a carbon-carbon bond with the monomer during the polymerization;
(iv) compounds capable of coordinating via a µ or η bond to the said compound comprising a transition metal;
the said polymer comprising one or more monomers Mi, the corresponding homopolymer of which exhibits a Tg of greater than or equal to 0°C, preferably of greater than or equal to 5°C and even better still of greater than or equal to 20°C; this or these monomers Mi being present, in the final polymer, in a minimum amount of 40% by weight, preferably in an amount of between 50 and 99% by weight and even better still in an amount of 60-90% by weight, with respect to the total weight of monomers;
and
the said polymer furthermore comprising one or more monomers Mj, the corresponding homopolymer of which exhibits a Tg of less than or equal to 0°C, preferably of less than or equal to -10°C and even better still of less than or equal to -15°C; this or these monomers Mj being present, in the final polymer, in a maximum amount of 60% by weight, preferably in an amount of between 1 to 50% by weight and even better still in an amount of 10-40% by weight, with respect to the total weight of monomers.

2. Composition according to claim 1, in which the monomers are chosen from:
- (meth)acrylic esters obtained from linear or branched aliphatic alcohols, preferably C₁-C₂₀ alcohols;
- C₁-C₂₀ (meth)acrylic esters comprising perfluoroalkyl units;
- C₁-C₂₀ (meth)acrylic esters comprising siloxane units;
- (meth)acrylamides obtained from linear, branched or cyclic aliphatic amines and/or from aromatic amines preferably comprising 1 to 20 carbon atoms, such as tert-butylacrylamide; or (meth)acrylamides, such as acrylamide, methacrylamide or di(C₁-C₄)alkyl(meth)-acrylamides;
- vinyl, allyl or methallyl esters obtained from linear or branched C₁-C₁₀ or cyclic C₁-C₆ aliphatic alcohols;
- vinylcaprolactam;
- optionally substituted styrene;
- their mixtures.

3. Composition according to one of the preceding claims, in which the initiator is chosen from the compounds of formula:
- R¹¹ₓR¹²_{y}R¹³_{z}C-(RX)ₜ, in which x, y and z represent an integer ranging from 0 to 4, t an integer ranging from 1 to 4, and x+y+z = 4-t;
- R¹³ₓC₆-(RX)_{y} (saturated ring with 6 carbons) , in which x represents an integer ranging from 7 to 11, y represents an integer ranging from 1 to 5, and x+y = 12;
- R¹³ₓC₆-(RX)_{y} (unsaturated ring with 6 carbons), in which x represents an integer ranging from 0 to 5, y represents an integer ranging from 1 to 6, and x+y = 6;
- -[-(R¹¹)(R¹²)(R¹³)C-(RX)-]ₙ, in which n is greater than or equal to 1; cyclic or linear;
- -[-(R¹²)ₓC₆(RX)_{y}-R¹¹-]ₙ, in which x represents an integer ranging from 0 to 6, y represents an integer ranging from 1 to 6 and n is greater than or equal to 1, with x+y = 4 or 6; cyclic or linear;
- -[-(R¹²)ₓC₆(RX)_{y}-R¹¹-]ₙ, in which x represents an integer ranging from 0 to 12, y represents an integer ranging from 1 to 12 and n is greater than or equal to 1, with x+y = 10 or 12; cyclic or linear;
- -[OSi(R¹¹)ₓ(RX)_{y}]ₙ, cyclic or linear, in which x and y represent an integer ranging from 0 to 2 and n is greater than or equal to 1, with x+y = 2;
- R¹¹N-X₂;
- (R¹¹)ₓP(O)_{y}-X₃₋ₓ, in which x and y represent integers ranging from 0 to 2 and x+y = 5;
- (R¹¹O)ₓP(O)_{y}-X₃₋ₓ, in which x and y represent integers ranging from 0 to 2 and x+y = 5;
- -[(R¹¹)ₜN_{z}P(O)ₓ(O-RX)_{y}-]ₙ, cyclic or linear, in which x represents an integer ranging from 0 to 4, y represents an integer ranging from 1 to 5, z represents an integer ranging from 0 to 2, t represents an integer ranging from 0 to 3 and n is greater than or equal to 1;
in which:
- R, R¹¹, R¹² and R¹³ represent, independently of one another, a hydrogen or halogen atom; a linear or branched alkyl radical having 1-20, preferably 1-10 and more preferably 1-6 carbon atoms; a cycloalkyl radical having 3-8 carbon atoms; a -C(=Y)R⁵, -C(=Y)NR⁶R⁷ or -R⁸₃Si radical; -COCl; -OH; -CN; an alkenyl or alkynyl radical having 2-20, preferably 2-6, carbon atoms; an oxiranyl or glycidyl radical or an alkylene or alkenylene radical substituted with an oxiranyl or a glycidyl; an aryl, heterocyclyl, aralkyl or aralkenyl radical; or an alkyl radical having 1-6 carbon atoms in which all or part of the hydrogen atoms are substituted either by halogen atoms, such as fluorine, chlorine or bromine, or by an alkoxy group having 1-4 carbon atoms or by an aryl, heterocyclyl, -C(=Y)R⁵, -C(=Y)NR⁶R⁷, oxiranyl or glycidyl radical;
- X represents a halogen atom, such as Cl, Br or I, or an -OR', -SR, -SeR, -OC(=O)R', -OP(=O)R', -OP(=O)(OR')₂, -OP(=O)OR', -O-NR'₂, -S-C(=S)NR'₂, -CN, -NC, -SCN, -CNS, -OCN, -CNO or -N₃ radical, in. which R' represents an alkyl radical having 1-20 carbon atoms which is optionally substituted by one or more halogen atoms, in particular fluorine and/or chlorine atoms, and R represents a linear or branched alkyl or aryl radical having 1-20, preferably 1-10, carbon atoms, it additionally being possible for the -NR'₂ group to represent a cyclic group, the two R' groups being joined so as to form a 5-, 6- or 7-membered heterocycle;
- Y represents O, S or NR⁸ (preferably O) ;
- R⁵ represents a linear or branched alkyl, alkylthio or alkoxy radical having 1-20 carbon atoms; an OH radical; an OM' radical with M' = alkali metal; an aryloxy radical or a heterocyclyloxy radical;
- R⁶ and R⁷ represent, independently of one another, H or a linear or branched alkyl radical having 1-20 carbon atoms; it being given that R⁶ and R⁷ can be joined to form an alkylene group having 2-7, preferably 2-5, carbon atoms;
- R⁸ represents H; a linear or branched alkyl radical having 1-20 carbon atoms or an aryl radical.

4. Composition according to one of the preceding claims, in which the initiator is chosen from:
- octa(2-isobutyrylbromide)-octa(tert-butyl)calix(8)arene,
- octa(2-propionylbromide)-octa(tert-butyl)calix(8)arene, and
- hexakis (a-bromomethyl)benzene.

5. Composition according to one of the preceding claims, in which the ligand is chosen from:
(i) the compounds of formula: R⁹-Z-(R¹⁴-Z)ₘ-R¹⁰ or R²⁰R²¹C[C(=Y)R⁵]
in which:
- R⁹ and R¹⁰ are, independently of one another, a hydrogen atom; a linear or branched alkyl radical having 1-20, preferably 1-10, carbon atoms; an aryl radical; a heterocyclyl radical; or an alkyl radical having 1-6 carbon atoms which is substituted with an alkoxy radical having 1-6 carbon atoms or a dialkylamino radical having 1-4 carbon atoms or a -C(=Y)R⁵ or -C(=Y)NR⁶R⁷ and/or YC(=Y)R⁸ radical, R⁵ to R⁸ and Y having the definitions given in Claim 3; it being given that R⁹ and R¹⁰ can be joined so as to form a saturated or unsaturated ring;
- R¹⁴ represents, independently of one another, a divalent group chosen from alkanediyls having 2-4 carbon atoms; alkenediyls having 2-4 carbon atoms; cycloalkanediyls having 3-8 carbon atoms; cycloalkenediyls having 3-8 carbon atoms; arenediyls and heterocyclylenes;
- Z represents 0, S, NR¹⁵ or PR¹⁵, with R¹⁵ representing H; a linear or branched alkyl radical having 1-20 carbon atoms; an aryl radical; a heterocyclyl radical; or an alkyl radical having 1-6 carbon atoms which is substituted with an alkoxy radical having 1-6 carbon atoms or a dialkylamino radical having 1-4 carbon atoms or a -C(=Y)R⁵ or -C(=Y)NR⁶R⁷ and/or YC(=Y)R⁸ radical (R⁵ to R⁸ and Y having the definitions given in Claim 3;
- m is between 0 and 6;
- R²⁰ and R²¹ are, independently of one another, a hydrogen atom; a halogen atom; a linear or branched alkyl radical having 1-20, preferably 1-10, carbon atoms; an aryl radical; or a heterocyclyl radical; it being given that R²⁰ and R²¹ can be joined so as to form a saturated or unsaturated ring; it being given that, in addition, each radical can be substituted with an alkyl radical having 1-6 carbon atoms, an alkoxy radical having 1-6 carbon atoms or an aryl radical;
(ii) carbon monoxide; optionally substituted porphyrins and porphycenes; optionally substituted ethylenediamine and propylenediamine; polyamines with tertiary amines, such as pentamethyldiethylenetriamine; aminoalcohols, such as aminoethanol and aminopropanol, which are optionally substituted; glycols, such as ethylene glycol or propylene glycol, which are optionally substituted; arenes, such as benzene, which are optionally substituted; optionally substituted cyclopentadiene; optionally substituted pyridines and bipyridines; acetonitrile; 1,10-phenanthroline; cryptands and crown ethers; or sparteine.

6. Composition according to one of the preceding claims, in which the polymer with a "star" structure makes it possible to obtain a film:
- having a modulus of elasticity (Young's modulus) of less than approximately 200 MPa, preferably of between 2 and 100 MPa, preferentially of between 5 and 80 MPa; and/or
- having a hardness of less than 110, preferably of between 1 and 70 and even better still of between 5 and 55.

7. Composition according to one of the preceding claims, in which the polymer with the "star" structure is present in an amount of between 1-50% by weight on a dry basis, with respect to the total weight of the composition, preferably between 1-40% by weight and preferentially between 5-35% by weight.

8. Composition according to one of the preceding claims, in which the polymer with a "star" structure is found in the medium in the dissolved form or in dispersion in an aqueous, organic, aqueous/organic and/or fatty phase.

9. Composition according to one of the preceding claims, which is provided in the form of oil-in-water or water-in-oil emulsions; of aqueous or oily dispersions or dispersions in a solvent medium; of aqueous or oily solutions or solutions in a solvent medium; in the thickened or gelled fluid, semisolid or soft paste form; or in the solid form, such as a stick.

10. Composition according to one of the preceding claims, which is provided in the form of a lipstick, foundation, face powder, eyeshadow, eyeliner or mascara.

11. Composition according to one of the preceding claims, which is provided in the form of a transfer-free makeup composition, in particular as transfer-free lipstick composition and/or as transfer-free foundation composition.

12. Process for the cosmetic treatment of keratinous substances, such as the skin, eyelashes, eyebrows, nails or lips, **characterized in that** it consists in applying, to the latter, a cosmetic composition according to one of Claims 1 to 11.

13. Use of at least one polymer as defined in Claim 1 in a cosmetic composition for reducing, indeed even eliminating, transfer of the composition film deposited.
